# EUROPEAN PATENT APPLICATION

(11) **EP 4 666 961 A1**
(43) Date of publication of application: **24.12.2025**
(21) Application number: 24756970.0
(22) Date of filing: 15.02.2024
(51) Int. Cl.: A61B 17/06, A61B 17/04, A61B 50/33

(54) **ARMED SUTURE SPOOLING DEVICE, ARMED SUTURE HOUSING TRAY, AND MEDICAL INSTRUMENT HOUSING TRAY**

(30) Priority: 17.02.2023 JP 2023023804; 25.05.2023 JP 2023086490; 26.05.2023 JP 2023087241; 26.05.2023 JP 2023087242; 07.09.2023 JP 2023145635
(71) Applicant: Mani, Inc., Tochigi 321-3231 (JP)
(72) Inventor: MATSUTANI, Kazuhiko, Utsunomiya-shi, Tochigi 321-3231 (JP); MURAKAMI, Etsuo, Utsunomiya-shi, Tochigi 321-3231 (JP)
(74) Representative: Gulde & Partner
(86) International application number: PCT/JP2024/005344
(87) International publication number: WO 2024/172130

(57) **Abstract**

Provided is an armed suture spooling device that facilitates a spooling operation with respect to a spool tray and allows for easy adjustment of the suture end position. A spool tray 10 for housing an armed suture, which is a suture with a suture needle attached thereto, comprises: a container part 20 having a bottom surface 21 and a side wall 25; and a sheet-shaped lid 30 that fits inside the side wall. A spooling device comprises an upper surface element 50, a lower surface element 60, a circling element 70, and connecting posts 80. The upper surface element and the lower surface element have a planar shape identical to that of the spool tray. The upper surface element includes an upper surface rotational portion 51 rotatable around an axis perpendicular to the lid portion. The lower surface element includes a lower surface rotational portion 61 rotatable around the same axis as that of the upper surface rotational portion. The circling element includes a claw portion 76 which is inserted through a gap between the lid portion and the side wall of the container portion into the container portion. The claw portion includes a thread-passing portion 77 at the tip-end thereof through which the suture is passed. The spool tray is sandwiched between the upper surface element from the upper surface side of the lid portion, and the lower surface element from the lower surface side of the bottom surface of the container portion. The connecting posts extend through the lid portion and the bottom surface of the container portion, with the ends of the connecting posts being connectable to post-insertion portions 55, 65 respectively provided in the upper surface element and the lower surface element. In a state in which the upper surface rotational portion and the lower surface rotational portion are pressed, the spool tray, the upper surface element, and the lower surface element can be integrally rotated, whereby the suture that has passed through the thread-passing portion can be wound inside the spool tray.

## Description

### [Technical Field]

This invention relates to a thread winding device that enables easy winding of needle-attached suture threads for medical use onto a thread winding tray for storing such needle-attached suture threads, a storage tray for needle-attached suture threads used for packaging needle-attached suture threads for medical use, and a medical device storage tray used for storing medical devices such as needle-attached suture threads.

### [Background Art]

### Background Art 1

Needle-attached suture threads for medical use, which are employed in surgical and dental procedures, are formed by attaching a suture thread to a straight or curved suture needle. Generally, the suture thread is inserted into a blind hole formed at the base end of the suture needle and is secured by crimping.

Needle-attached suture threads stored in a case, such as a tray, are sterilized and the entire case is sealed in a packaging bag and provided as a commercial product. The packaging bag, made of a bacteria-impermeable film or other material, hermetically encloses the sterilized case by sealing around its periphery. The packaging bag is then opened during surgery, the suture needle is grasped with a needle holder, the suture thread is pulled out from an opening formed in the lid or the like, and the suture thread is used for suturing.

Cases in which needle-attached suture threads are stored are generally made of paper, resin, or trays in which a resin container is covered with a paper lid (see, for example, Patent Document 1). The suture threads are stored in a wound state inside the case in a circular shape, oval shape, or figure-eight pattern. Therefore, when the suture needle is picked up with a needle holder and the suture thread is pulled out, the suture thread needs to be taken out smoothly without becoming tangled. Accordingly, the method of winding the suture thread is important.

The method of winding needle-attached suture thread may be performed either manually or by machine. In manual winding, for example, the suture thread is wound around two rods in a figure-eight pattern, then removed from the rods and stored in a case. In this case, the thread winding operator repeatedly winds the suture thread around the two rods in the same posture. On the other hand, a machine may be used to automatically wind the suture thread onto a thread winding tray including a plastic container part covered with a paper or other lid (see, for example, Patent Documents 2 and 3).

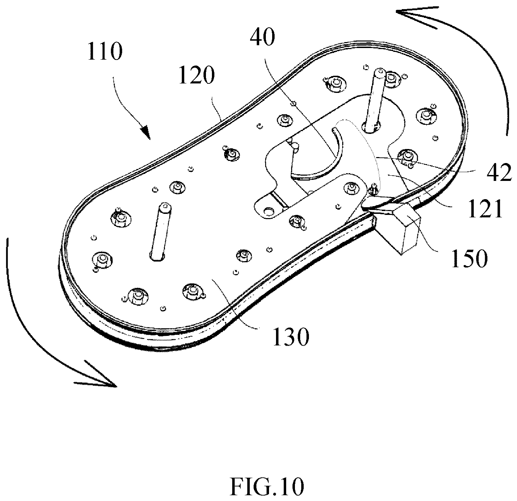

Fig. 10 illustrates the conventional method of winding suture threads on a thread winding tray. In this case, a needle-attached suture thread 40 is retained in the suture needle retaining portion provided in the container part 120, a claw portion of a thread insertion member 150, which guides a suture thread 42 near a bottom surface 121 of the container part 120, is inserted between the container part 120 and the lid 130. As the thread winding tray 110 is rotated relative to the thread insertion member 150, the thread insertion member 150 moves along the outer periphery of the thread winding tray 110, thereby winding the suture thread 42 inside the thread winding tray 110. However, the setup of the thread winding tray 110 and the needle-attached suture thread 40 basically needs to be perfomed manually. Fully automating the process would require a substantial cost and a large-scale machine. In addition, if the suture threads are thin and easily broken, they need to be handled carefully, and in some cases, it is better to perform the thread winding by hand than by machine.

If the packaging bag is sealed with suture thread protruding from the thread winding tray, the protruding suture thread may become caught in the sealed area, resulting in problems such as failure to maintain sterilization safety. When using a suture thread with high straightness, such as a monofilament thread, the suture thread wound in a curved pattern tends to protrude from the winding tray as the suture thread attempts to return to a straight line. Therefore, it is common practice to first attach the lid to the container part and then carefully perform the winding operation while preventing the suture thread from protruding.

The tip of the suture thread tends to protrude from the thread winding tray most easily, especially at curved portions of the thread winding tray or at the opening of the lid, where the curved suture thread tends to protrude as it attempts to stretch into a straight line. Therefore, it is desirable to perform the thread winding opertion so that the tip of the suture thread is positioned on a straight portion of the thread winding tray where it is difficult for the suture thread to protrude. However, there are many factors that affect the position of the tip of the suture thread, such as the length of the suture thread, the shape of the thread winding tray, and the tightness of the winding, making it complicated to adjust the tip position of the thread. In addition, when the thread winding operation is performed by machine, it is difficult to ensure that the tip of the suture thread remains inside the container part, and it is necessary to make the tray rotate at a high speed, which then makes it difficult to wind thin suture threads by machine.

### Background Art 2

Needle-attached suture threads for medical use, which are employed in surgical and dental procedures, are formed by attaching a suture thread to a straight or curved suture needle. Generally, the suture thread is inserted into a blind hole formed at the base end of the suture needle and is secured by crimping.

Needle-attached suture threads stored in a case such as a storage tray are sterilized and the entire case is sealed inside a packaging bag and provided as a commercial product. The packaging bag, made of a bacteria-impermeable film or other material, hermetically encloses the sterilized case by sealing around its periphery. The packaging bag is then opened during surgery, the suture needle is grasped with a needle holder, the suture thread is pulled out from the case, and the suture thread is used for suturing.

Cases in which needle-attached suture threads are stored are generally made of paper, resin, or storage trays in which a resin container is joined with a paper lid member. The suture threads are stored in a wound state inside the case in a circular shape, oval shape, or figure-eight pattern.

The method of winding suture thread may be performed either manually or by machine. In the case of manual work, for example, the suture thread is wound on two rods so that the suture thread forms a figure-eight pattern and stored in a storage tray. In manual winding, for example, the suture thread is wound around two rods in a figure-eight pattern, stored in a storage tray. On the other hand, a machine may be used to wind the thread directly into the storage tray including a plastic container member covered with a paper lid member.

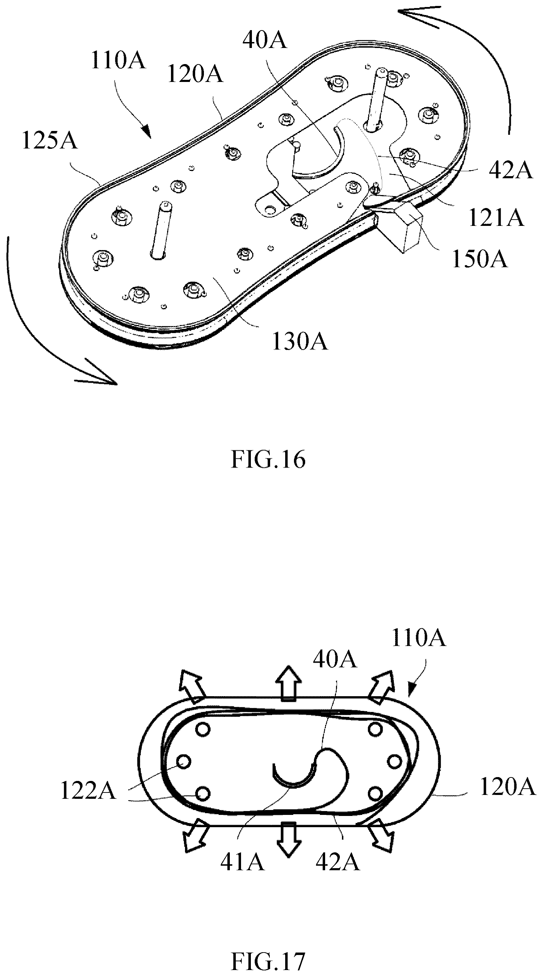

Fig. 16 illustrates a method of winding the suture thread inside the storage tray. In this case, a needle-attached suture thread 40A is retained to the bottom surface 121A of container member 120A, the tip of thread insertion jig 150A, which guides a suture thread 42A near the bottom surface 121A of container member 120A, is inserted between the container member 120A and lid member 130A. As the storage tray 110A is rotated relative to the thread insertion jig 150A, the thread insertion jig 150A moves along the outer periphery of the storage tray 110A, thereby winding the suture thread 42A inside the storage tray 110A.

Fig. 17 illustrates the state of needle-attached suture thread in the storage tray. In this illustration, a suture thread 42A is wound in an elliptical shape around the outside of mating posts 122A used to join the lid member in the container member 120A. When using a suture thread 42A with high straightness, such as a monofilament thread, the sture thread 42A wound in a curved pattern tends to protrude from the storage tray110A as the thread wound 42A attempts to return to a straight line. Therefore, it is common practice to first attach the lid member to the container part 120A and then carefully perform the winding operation. While the tip of the suture thread 42A naturally tends to protrude, when the suture thread 42A is wound in an elliptical shape, as shown in Fig. 17, for example, the suture thread 42A tends to form a straight line at the left and right positions of the arc portions, so it expands and protrudes easily especially near the straight portions of the elliptical shape. In addition, since the straight portions of the storage tray 110A and the suture thread 42A are parallel to each other, the suture thread 42A tends to protrude from the storage tray 110A when vibrations are applied in this state.

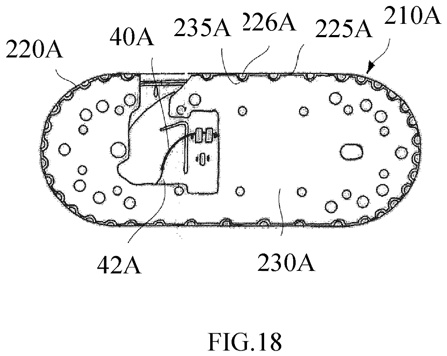

If the packaging bag is sealed with the suture thread 42A protruding from the storage tray 110A, the protruding suture thread may become caught in the sealing area, resulting in problems such as failure to guarantee sterilization safety. Fig. 18 shows a plan view of a conventional storage tray for preventing protrusion of suture thread as shown in Patent Document 1. This storage tray 210A includes a plastic container member 220A and a paper lid member 230A attached thereto. A plurality of projection-shaped ribs 226A are provided inside a side wall 225A of the container member 220A. Notches 235A shaped to fit the ribs 226A are provided along the outer periphery of the lid member 230A. In other words, when the lid member 230A is attached to the container member 220A, the ribs 236A fit into the positions of the notches 235A of the lid member 230A. Thus, when the notches 235A are provided on the outer periphery of the lid member 230A, a corrugated edge portion is formed along the outer periphery of the lid member 230A in a planar direction, thereby producing the effect of preventing the suture thread 42A of the needle-attached suture thread 40A from protruding from the storage tray 210A. Specifically, even if the tip of the suture thread 42A protrudes from a gap between the container member 220A and the lid member 230A, the amount of protrusion from the tip can be suppressed by having the suture thread 42A caught in the notches 235A. However, when performing the thread winding operation, the thread insertion jig 150A or suture thread 42A may easily become caught in the notches 235A, making it difficult to perform the thread winding operation smoothly.

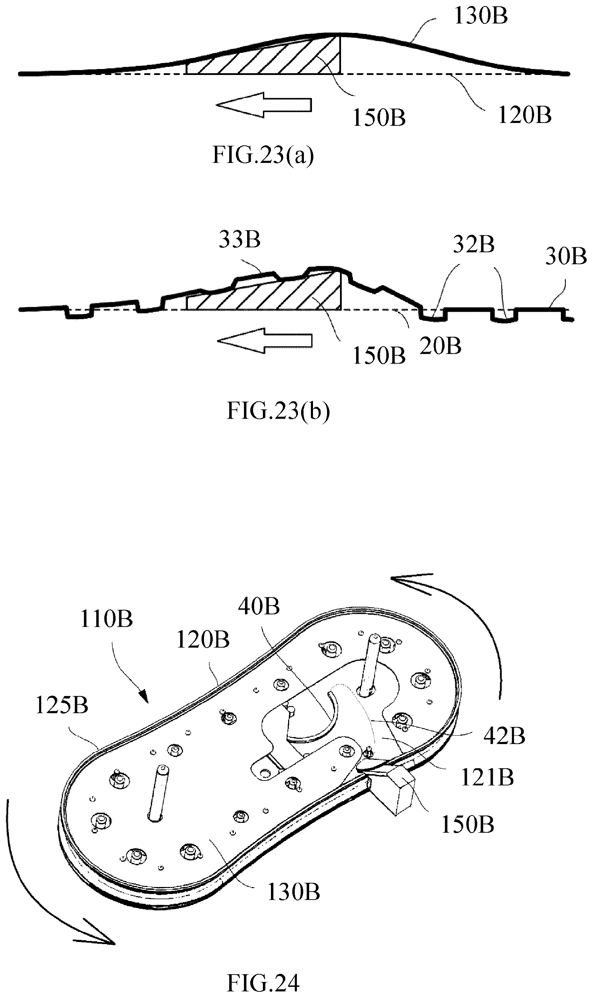

Fig. 24 illustrates a method of winding the suture thread inside the storage tray. In this case, a suture needle of needle-attached suture thread 40B is retained to the bottom surface 121B of container member 120B, the tip of thread insertion jig 150B, which guides the suture thread 42B near bottom surface 121B of container member 120B, is inserted from between the container member 120B and lid member 130B. As the storage tray 110B is rotated relative to the thread insertion jig 150B, the thread insertion jig 150B moves along the outer periphery of the storage tray 110B, thereby winding the suture thread 42B inside the storage tray 110B.

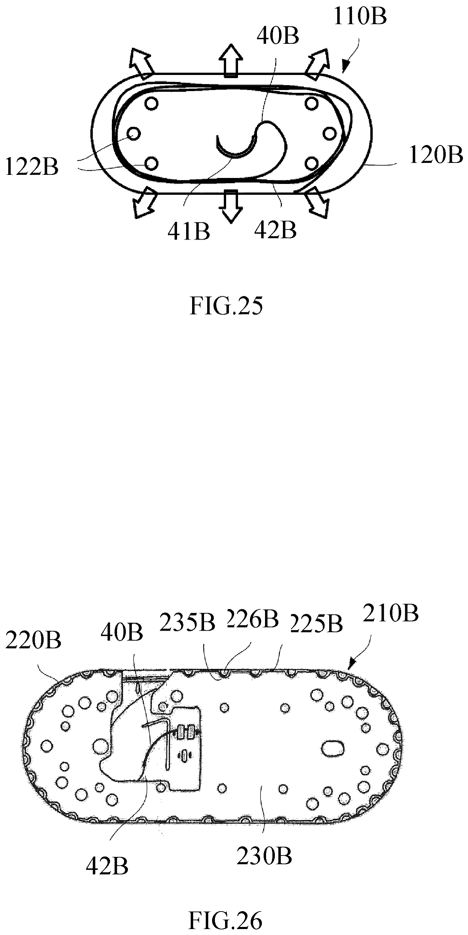

Fig. 25 illustrates the state of needle-attached suture thread in the storage tray. In this illustration, a suture thread 42B is wound in an elliptical shape around the outside of the mating posts 122B used to join the lid member in the container member 120B. When using a suture thread 42B with high straightness, such as a monofilament thread, the suture thread 42B wound in a curved pattern tends to protrude from the storage tray110B as the thread wound 42B attempts to return to a straight line. Therefore, it is common practice to first attach the lid member to the container part 120B and then carefully perform the winding operation. While the tip of the suture thread 42B tends to protrude the most, when the suture thread 42B is wound in an elliptical shap, as shown in Fig. 25, for example, the suture thread 42B tries to form a straight line at the left and right positions of the arc portions, so it may spread outward and protrude near the straight portion of the elliptical shape. In addition, since the straight portions of the storage tray 110B and the suture thread 42B are parallel to each other, the suture thread 42B tends to protrude from the storage tray 110B when vibrations are applied in this state.

If the packaging bag is sealed with suture thread 42B protruding from the storage tray 110B, the protruding suture thread may become caught in the sealing area, resulting in problems such as failure to guarantee sterilization safety. Fig. 26 shows a plan view of a conventional storage tray for preventing protrusion of suture thread as shown in Patent Document 1. This storage tray 210B includes a plastic container member 220B and a pepar lid member 230B attached thereto. A plurality of projection-shaped ribs 226B are provided inside an outer wall 225B of the container member 220B. Notches 235 B shaped to fit the ribs 226B are provided along the edge of the lid member 230B. In other words, when the lid member 230B is attached to the container member 220B, the ribs 236B fit into the position of the notches 235B of the lid member 230B. Thus, when the notches 235B are provided on the edge of the lid member 230B, concave-convex portions are formed along the edge of the lid member 230B in the planar direction, thereby producing the effect of preventing the stored suture thread 42B from protruding from the storage tray 210B.

Specifically, even if the tip of the suture thread 40B protrudes from a gap between the container member 220B and lid member 230B, the amount of protrusion from the tip can be suppressed by having the suture thread 42B caught in the notches 235B. However, when the thread insertion jig 150B is inserted between the lid member 230B and the container member 220B for the thread winding operation, the tip of the suture thread 42B remains released from the thread insertion jig 150B until the thread insertion jig 150B is removed from the storage tray 210B after the thread winding operation is completed. As a result, the tip of the suture thread 42B may easily protrude from a gap between the lid member 230B and the container member 220B caused by the insertion of the suture thread 42B. In addition, the thread insertion jig 150B or suture thread 42B may easily become caught in the notches 235B, making it difficult to perform the thread winding operation smoothly.

### Background Art 3

Needle-attached suture threads for medical use, which are employed in surgical and dental procedures, are formed by attaching a suture thread to a straight or curved suture needle. Generally, the suture thread is inserted into a blind hole formed at the base end of the suture needle and is secured by crimping. Suture threads may be classified into various types based on material and shape, and examples include absorbable threads and barbed suture threads.

Medical devices, such as needle-attached suture threads or the like, stored in storage trays or the like are sterilized, and the entire storage tray is sealed inside a packaging bag and provided as a commercial product. The packaging bag, which is made of a bacteria-impermeable film or other material, hermetically encloses the sterilized storage tray by sealing around its periphery. The packaging bag is then opened during surgery, and in the case of needle-attached suture threads, a suture needle is grasped with a needle holder, the suture thread is pulled out of from the storage tray, and the suture thread is used for suturing.

Cases in which needle-attached suture threads are stored are generally made of paper or resin cases, or storage trays in which a plastic container is attached with a lid member. The suture threads are stored in a wound state in a circular shape, oval shape, or figure-eight pattern.

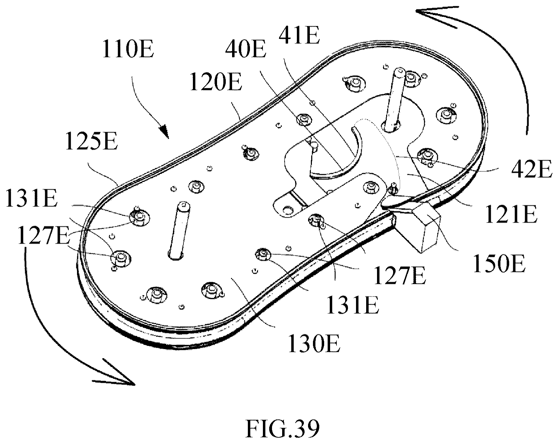

Fig. 39 illustrates a conventional method of winding the suture thread onto a storage tray. The storage tray 110E illustrated is a container member 120E joined with a paper or plastic lid member 130E. The method of joining the container member 120E and the lid member 130E is performed by passing a plurality of mating posts 127E rising from the bottom surface 121E of the container member 120E through mating holes 131E formed in the lid member 130E. By making the tip of the mating posts 127E slightly larger than the mating holes 131E, it is possible to prevent the attached lid member 130E from coming off. In this case, the larger the tip portion is, the harder it is for the lid member 130E to come off, but if the tip portion is too large, it becomes difficult to attach the lid member 130E. Therefore, after passing the mating posts 127E with a smaller diameter than the mating holes 131E through the mating holes 131E, the tip portion of the mating posts 127E may be deformed so that it is larger than the mating holes 131E to prevent the lid member 130E from coming off. This method of joining the container member 120E and the lid member 130E can be securely joined, but the joining work is complicated and the storage space becomes small because the mating posts 127E are inside the storage container.

When performing machine thread winding on the storage tray 110E shown in Fig. 39, the suture needle of needle-attached suture thread 40E is retained to the bottom surface 121E of container member 120E, the tip of thread insertion jig 150E, which guides a suture thread 42E near the bottom surface 121E of container member 120E, is inserted between the container member 120E and lid member 130E. As the storage tray 110E is rotated relative to the thread insertion jig 150E, the thread insertion jig 150E moves along the outer periphery of the storage tray 110E, thereby winding and storing the suture thread 42E around the mating posts127E.

In this type of machine thread winding operation, it is particularly important to ensure that the tip of suture thread 42E separates from the thread insertion jig 150E and protrudes outside the storage tray 110E in the final stage of thread winding operation. If the packaging bag is sealed with the suture thread 42E protruding from the storage tray 110E, the suture thread protruding from the storage tray 110E may become caught in the sealing area of the packaging bag, resulting in problems such as failure to guarantee sterilization safety.

There are also storage trays in which suture thread is wound into a figure-eight pattern or similar shape without using the thread insertion jig 150E as shown in Fig. 39 (see, for example, Patent Document 4). Fig. 40 shows an inside view of the upper lid member with the suture thread wound in a figure-eight pattern. The upper lid member 310E illustrated is joined with the lower lid member (not shown) to form a storage tray for storing needle-attached suture threads.

In this storage tray, the suture needle 41E of needle-attached suture thread 40E is retained to a needle retaining portion 320E provided in the upper lid member 310E, and suture thread 42E is wound and stored in a figure-eight pattern. The upper lid member 310E is provided with an inner pressing portion 340E and an outer pressing portion 350E, and the suture thread 42E is wound in a figure-eight pattern by being passed through a passage between the inner pressing portion 340E and the outer pressing portion 350E. This configuration is considered to be highly effective in preventing the suture thread 42E from protruding from the storage tray. However, the structure of the upper lid member 310E is complex, and the process of winding the suture thread 42E in a figure-eight pattern in a defined path is complicated.

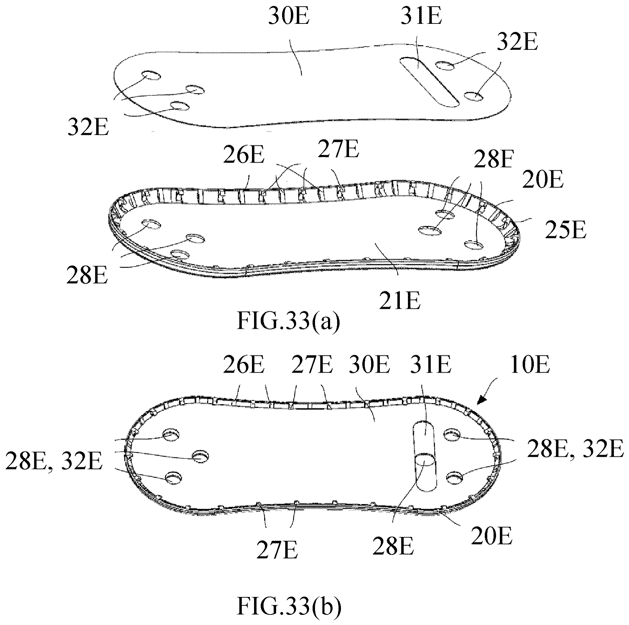

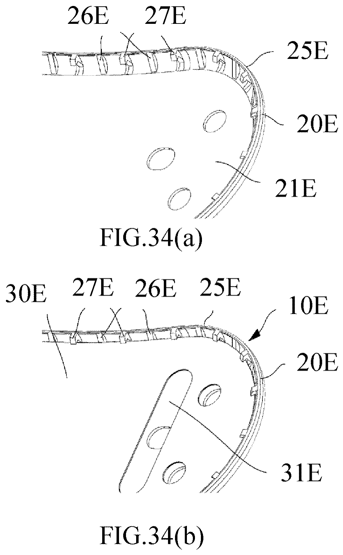

In addition, when using storage trays such as those shown in Figs. 33 and 34 to store barbed suture threads, the barbs may become caught when the suture threads are pulled out. Therefore, storage trays designed for pulling out the suture thread are not suitable for storing barbed suture threads. Furthermore, when the lid member 130E is attached using the mating posts 127E or when the internal structure is complicated as shown in Fig. 34, the needle-attached suture threads 40E need to be wound and stored into a specific shape, but if the storage tray has a larger storage space, it is possible to lightly wind the needle-attached suture thread or to store medical devices other than needle-attached suture threads.

Patent Document 5 discloses an automatic packaging apparatus for needles with suture threads, in which the base, outer wall, inner wall, and fingers of the automatic packaging apparatus for needles with suture threads define a hollow peripheral groove for accommodating a suture winding loop, and in which the fingers are bendable and have a characteristic of springing back essentially to their original horizontal position when released, thereby allowing easy insertion of the suture thread 80C in the open position and facilitating storage of the suture thread loop within the hollow peripheral groove in the closed position.

### [RELATED ART DOCUMENTS]

### [PATENT DOCUMETNTS]

[Patent Document 1] U.S. Patent No. 10835237
[Patent Document 2] U.S. Patent No. 10981686
[Patent Document 3] JP Patent No. 3688326
[Patent Document 4] CN Patent Publication No. 201389047
[Patent Document 5] U.S. Patent No. 5664404

### [Disclosure of the Invention]

### [Problems to be Solved by the Invention]

In view of these circumstances, the objects of the present invention are:
(1) to provide a thread winding device for needle-attached suture thread that enables easy thread winding regardless of the size of the suture thread and furthermore enables easy adjustment of the tip position of the suture thread to prevent protrusion of the thread end;
(2) to provide a storage tray for needle-attached suture thread that has a configuration that does not interfere with the thread winding operation and prevents protrusion of the stored suture thread;
(3) to provide a storage tray for needle-attached suture thread that can prevent protrusion of the suture thread during winding and after storing and enable smooth thread winding operation;
(4) to provide a storage tray for needle-attached suture thread that can prevent protrusion of the suture thread during winding and after storing without using a lid member and that enables quick thread winding operation; and
(5) to provide a medical device storage tray that can store medical devices such as needle-attached suture threads, and that has a structure in which the lid member and the container member are easily joined as well as the lid member is resistant to unintentional removal, and in which sufficient storage space can be secured.

### [Means of Solving the Problems]

(1) A thread winding device according to the presen invention is a thread winding device for storing needle-attached suture threads in a thread winding tray with a container part and an upper member, including:
   a lower surface member supporting the container part from a bottom surface thereof; and
   a circumferential member capable of moving around the lower surface member along a side surface of the lower surface member,
   in which the lower surface member includes a guide portion on an outer periphery thereof,
   the circumferential member includes: a claw portion insertable between the upper member and the container part; and an engagement portion engageable with the guide portion, and
   the circumferential member is engageable with the guide portion with the claw portion inserted into an interior of the container part.

The thread winding device may include an upper surface member capable of pressing the upper member from above. The upper surface member may include joining portions. Coupling members may be attached to all or part of the joining portions. The upper member may inlcude through-portions allowing all the coupling members to pass through, individually.

The engagement portion may include contact points inside the engagement portion. The contact points may being capable of contacting the guide portion. The contact points are capable of contacting the guide portion at three or more locations. At least one of the contact points may be a roller.

(2)
A storage tray according to the present invention is a storage tray for winding and storing needle-attached suture threads, including:
a container member including a bottom surface and a side wall,
in which an inner surface of the side wall is provided with a plurality of ribs, each rib being connected to the bottom surface and the side wall and projecting toward an inside of the container member, and
a top surface of each rib is lowered toward the inside of the container member.

The storage tray may include a lid member capable of being joined to the container member. The top surface of each rib may be a straight line, a curve, or a combination of a straight line and a curve, being highest at a point connected to the side surface of the container member and lowest at a point connected to the bottom surface. The lid member may be in contact with the top surface of each rib when the lid member and the container member are joined.

At a position between adjacent ribs, the lid member may include one or more convex portions toward the bottom surface of the container member. The one or more convex portions may be a plurality of convex portions, thereby forming a series of protrusions and recesses in a thickness direction along an edge of the lid member.

(3)
A storage tray according to the present invention is a storage tray for winding and storing a needle-attached suture thread, inlcuding:
a container member including a bottom surface and a side wall; and
a lid member capable of being joined to the container member,
in which the lid member includes a plurality of groove portions extending inward from an edge of the lid member, forming a series of protrusions and recesses in a thickness direction along the edge of the lid member.

The lid member may include the groove portions in a flat plate made of resin, whereby a direction along the edge of the lid member has lower rigidity than a flat plate portion of the lid member, and a direction from the edge toward an interior of the lid member has higher rigidity than the flat plate portion of the lid member.

An inner surface of the side wall may be provided with a plurality of ribs, each rib being connected to the bottom surface and the side wall and projecting toward an inside of the container member. Each groove portion formed in the lid member may be located between adjacent ribs.

A storage tray according to the present invention is a storage tray for winding and storing needle-attached suture threads, including:
a bottom surface;
an outer wall provided on the bottom surface; and
an inner wall provided on the bottom surface and located inward of the outer wall,
in which the outer wall includes an outer wall surface facing the inner wall,
the inner wall includes an inner wall surface facing the outer wall,
the outer wall surface of the outer wall is provided with a plurality of ribs, each rib being connected to the bottom surface and the outer wall surface and projecting toward the inner wall,
the inner wall includes a plurality of inner wall arms extending toward the outer wall, and
a sum of height of the rib from the outer wall surface and height of the inner wall arm adjacent the rib from the inner wall surface is longer than a wall-to-wall distance between the outer wall surface and the inner wall surface.

A top surface of the rib may be lowered toward the inner wall.

The top surface of the rib may be a straight line, a curve, or a combination of a straight line and a curve, being highest at a point connected to the outer wall surface and lowest at a point connected to the bottom surface.

A tip end of the inner wall arm may be located between the outer wall surface and a position corresponding to a height of each rib in plan view.

A tip end of the inner wall arm may be located closer to the bottom surace than a base end thereof.

The bottom surface may include a plurality of slits.

Each of the plurality of slits may be located under each of the plurality of inner wall arms in plan view.

Each of the plurality of slits may include a first slit region and a second slit region located between the inner wall arms respectively in plan view.

A width of each inner wall arm may be shorter than a sum of width of the first slit area and width of the second slit area.

(4)
A storage tray according to the present invention is a storage tray for winding and storing needle-attached suture threads, including:
a bottom surface;
an outer wall provided on the bottom surface; and
an inner wall provided on the bottom surface and located inward of the outer wall, in which the outer wall includes a plurality of outer wall arms extending toward the inner wall,
the inner wall includes a plurality of inner wall arms extending toward the outer wall,
the outer wall includes an outer wall surface facing the inner wall,
the inner wall includes an inner wall surface facing the outer wall, and
a sum of height of the outer wall arm from the outer wall surface and height of the inner wall arm from the inner wall surface is shorter than a wall-to-wall distance between the outer wall surface and the inner wall surface.

The bottom surface may include a plurality of slits. Each of the plurality of slits may be located under each of the plurality of outer wall arms and the plurality of inner wall arms in plan view. A width of each of the plurality of slits may be wider than the width of the corresponding outer and inner wall arms.

Each of the plurality of outer wall arms may be arranged alternately or partially alternately with each of the plurality of inner wall arms.

Each of the plurality of outer wall arms or each of the plurality of inner wall arms may be a Y-shaped arm.

(5)
A medical device storage tray according to the present invention include:
a container member including a bottom surface and a side wall, and
a lid member shaped to fit inside the side wall,
in which the inner surface of the side wall is provided with hook portions and ribs projecting toward an inside of the container member,
each rib and each hook portion are alternately repeated around an inner circumference of the container member, and
the lid member fits between the ribs and the hook portions when the lid member is attached to the container member.

Each rib may include an inclined portion downward toward the inside of the container member. The lid member may fit between the inclined portion and each hook portion when the lid member is attached to the container member. An outer peripheral shape of the medical device storage tray may include a series of alternating right and left curves.

### [Effects of the Invention]

By using the suture thread winding device for storing needle-attached suture threads, the suture thread can be easily wound onto the thread winding tray regardless of the size of the suture thread. Furthermore, by appropriately selecting the installation position of the thread winding pin, the tip position of the suture thread can be easily adjusted.

According to the suture thread storage tray for winding and storing needle-attached suture threads, the configuration does not interfere with the thread winding operation and can prevent the protrusion of stored suture thread from the storage tray.

According to the invention, the groove portions formed in the lid member can prevent protrusion of the suture thread from the storage tray during winding and after storing. In addition, the concave-convex portions formed along the edge of the lid member can reduce the rigidity in the direction along the edge of the lid member, thereby producing the effect of facilitating the thread winding operation.

According to the present invention, suture thread protrusion can be prevented during winding and after storage without the use of a lid member, thus enabling rapid thread winding operations.

According to the present invention, the container member is joined to the lid member using ribs and hook portions provided on the side wall of the container member, which allows the interior of the storage tray to be used more effectively, thereby enabling storage of needle-attached suture threads and medical instruments of various types. Furthermore, the lid member can be easily joined with the container member. By each rib including an inclined portion downward toward the inside of the container member and the outer peripheral shape of the medical device storage tray including a series of alternating right and left curves, unintentional detachment of the lid member from the container member can be inhibited.

### [Brief Description of the Drawings]

Fig. 1 shows two different examples of an upper member and a container part of a thread winding tray used in the present, in a separated state.
Fig. 2 shows two examples of the upper member and the container part of the thread winding tray used in the present invention, in a joined state.
Fig. 3 shows an example of a thread winding device and the thread winding tray according to the present invention, in a separated state.
Fig. 4 shows an example of the thread winding device and the thread winding tray according to the present invention, in which the thread winding tray is mounted on a lower surface member.
Fig. 5 shows an underside view of an example of the thread winding device and the thread winding tray according to the present invention, in which the thread winding tray is mounted on the lower surface member.
Fig. 6 shows an example of the thread winding device according to the present invention, in which all components of the thread winding device are set in the thread winding tray.
Fig. 7 illustrates the movement of the circumferential member by the number of contact points.
Fig. 8 illustrates the relationship between the shape of the guide portion and the contact points of the circumferential member.
Fig. 9 illustrates the location of the thread winding pin.
Fig. 10 illustrates the conventional method of winding suture threads on a thread winding tray.
Fig. 11 shows perspective views of the storage tray of the present invention, where (a) shows the state before the lid member is attached, and (b) shows the state after the lid member is attached.
Fig. 12 shows a diagram of ribs provided on the container member.
Fig. 13 shows simplified cross-sectional views of the positional relationship between the lid member and the rib, where (a) shows a case in which the rib is composed of an inclined surface and a vertical surface, (b) shows a case in which the top surface of the rib is curved, and (c) shows a case in which the top surface of the rib is straight.
Fig. 14 shows diagrams of one or more convex portions provided on the lid member, where (a) and (b) show diagrams of a side wall in cross-sectional orientation, and (c) shows an example of an A-A cross-sectional view.
Figs. 15 (a) and (d) show a perspective view of an example of the lid member with convex portions and an enlarged view thereof, respectively.
Fig. 16 illustrates a method of winding the suture thread inside the storage tray.
Fig. 17 illustrates the state of needle-attached suture thread in the storage tray.
Fig. 18 shows a plan view of a conventional storage tray for preventing protrusion of suture thread.
Fig. 19 shows an exploded view of the lid and container members of the storage tray of the present invention.
Fig. 20 shows diagrams of the storage tray of the present invention, where (a) shows a perspective view thereof, and (b) and (c) show enlarged views of the edge of the lid member.
Fig. 21 shows simplified cross-sectional views of the positional relationship between the lid member and the ribs, where (a) shows a diagram of a side wall in cross-sectional orientation, and (b) shows an example of an A-A cross-sectional view.
Fig. 22 shows an enlarged view of the rib, in which the top surface of the rib is curved.
Fig. 23 shows illustrations comparing the shape of the lid member during the thread winding operation, where (a) shows a case without concave-convex portions, and (b) shows a case with concave-convex portions.
Fig. 24 illustrates a method of winding the suture thread inside the storage tray.
Fig. 25 illustrates the state of needle-attached suture thread in the storage tray.
Fig. 26 shows a plan view of a conventional storage tray for preventing protrusion of suture thread.
Fig. 27 shows a plan perspective view of the storage tray 1C of the present invention.
Fig. 28 shows partial plan views of the storage tray 1C, schematically illustrating the storage of the suture thread 26C in the storage tray 1C according to the present invention, where (a) shows the state before movement of the thread insertion jig 30C, (b) shows the state after the movement of the thread insertion jig 30C, and (c) shows a further movement of the thread insertion jig 30C beyond that shown in (b).
Fig. 29 shows schematic plan views of examples of outer wall arms in the storage tray 1C according to the present invention, where (a) shows a vertical outer wall arm 11C', and (b) shows an inclined outer wall arm 11C".
Fig. 30 shows schematic plan views of examples of arms in the storage tray 1C of the present invention, where (a) shows a Y-shaped outer wall arm, and (b) shows a Y-shaped inner wall arm.
Fig. 31 shows a plan perspective view of another example of the storage tray 1D of the present invention.
Fig. 32 shows a partial plan view of another example of the storage tray 1D of the present invetion.
Fig. 33 shows perspective views of the storage tray of the present invention, where (a) shows an exploded view of the lid member and the container member, and (b) shows a diagram of the lid member and the container member in a joined state.
Fig. 34 shows an enlarged view of the storage tray of the present invention, where (a) shows a diagram of an edge portion of the container member, and (b) shows a diagram of the edge portion where the lid member and the container member are joined.
Fig. 35 shows simplified cross-sectional views and A-A (B-B) sectional views of the container member with the lid member joined, where (a) shows a case in which the top surface of the rib is an inclined surface, and (b) shows a case in which the top surface of the rib is a curved surface.
Fig. 36 shows an example of a lid member with a tab.
Fig. 37 illustrates how to use the thread winding jig.
Fig. 38 illustrates a method of winding the suture thread in a figure-eight pattern using the thread winding jig.
Fig. 39 illustrates a conventional method of winding the suture thread around a storage tray.

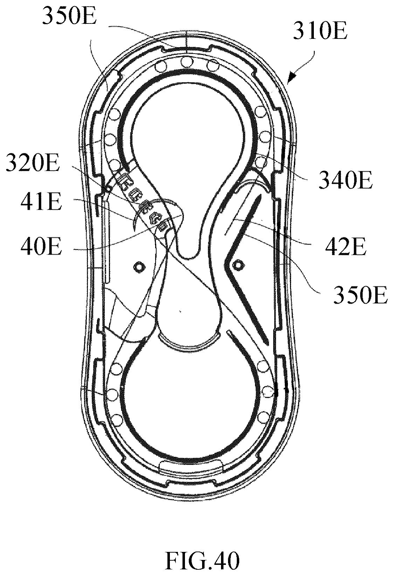

Fig. 40 shows an inside view of the upper lid member with the suture thread wound in a figure-eight pattern.

### [Description of Embodiments]

The following is a description of the invention with reference to the accompanying drawings.

### 1 Thread winding tray for storing needle-attached suture threads for medical use

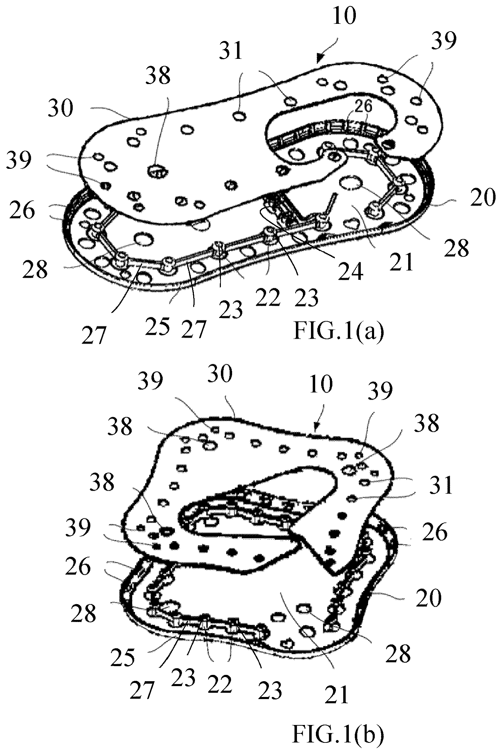

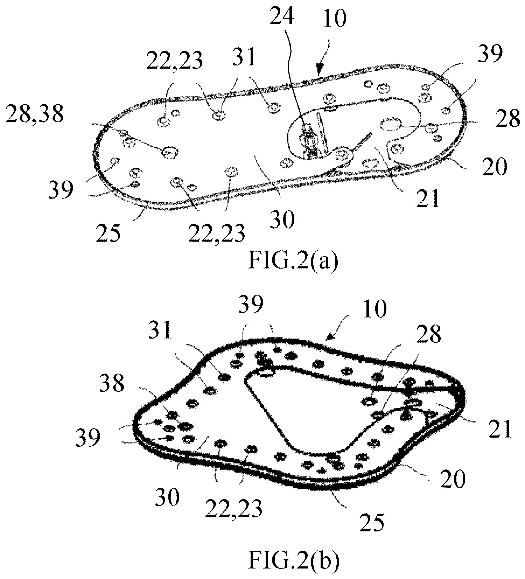

Fig. 1 shows two different examples of an upper member and a container part of a thread winding tray used in the present invention, in a separated state. Fig. 2 shows two examples of the upper member and the container part of the thread winding tray used in the present invention, in a joined state. These thread winding trays 10 are trays for storing needle-attached suture threads, and after storing the suture threads, each entire tray is wrapped and sealed with a bacteria-impermeable film or other material.

The basic configuration of the thread winding tray 10 has the container part 20 and the upper member. The upper member is a component or group of components that restrains the stored suture threads from protruding from the tray and may be a lid 30, or lid members (e.g., lid members 30A, 130A, 230A, 30B, 130B and 230B) or arms (e.g., outer wall arm 11C, inner wall arm 12C and inner wall arm 12D), which are described below. The overall planar shape of the thread winding tray 10 may be a rectangular shape, a rectangular shape with rounded corners, a circular shape, an oval shape, an elliptical shape which has an outer periphery consisting of arcs and straight lines or constricted straight portions, or a rectangular shape with constricted straight portions and rounded corners. The size of the thread winding tray 10 may be small enough to fit in the hand. The elliptical shape which has an outer periphery consisting of arcs and constricted straight portions refers to a shape such as that shown in Figs. 1(a) and 2(a), and is, for example, similar to the shape of a snowboard. In other words, when the thread winding tray 10 is placed horizontally, the outer peripheral shape is a circular arc, such as a semi-circle, on each of the left and right sides, and the upper and lower portions connecting the arcs are curves that are constricted so that they are close to each other. The rectangular shape with the constricted straight portions and the rounded corners refers to a shape such as that shown in Figs. 1(b) and 2(b). These shapes are characterized by the fact that when the outer periphery of the thread winding tray 10 is traced, there are almost no straight lines, and the right and left curves are alternated to form an annular shape. In the thread winding trays 10 as shown in Figs. 1(b) and 2(b), it is possible to store several needle-attached suture threads together.

The container part 20 is basically made of resin and has a bottom surface 21, a side wall 25, a plurality of substantially cylindrical mating posts 22 projecting from the bottom surface 21, and a needle retaining portion 24. The mating posts 22 are provided mainly for attaching the lid 30, and although it depends on the size and shape of the container, generally from several to approximately twenty posts are provided between the center of gravity of the container part 20 and the side wall 25, and the arrangement of the installation positions of the mating posts 22 forms an annular shape. The suture thread is basically wound around the outside of these annularly arranged mating posts 22. The height of the side wall 25 is less than 1 cm, as long as it is high enough to wind the suture thread.

Since the needle retaining portion 24 is only required to be able to hook and secure a suture needle in the thread winding operation, it may be the needle retaining portion 24 integrally formed with the container part 20, or it may be a separate sponge-like material that can be affixed on the bottom surface 21 to pierce and secure the suture needle's tip.

The lid 30 is about the same size as the container part 20 or fits inside the side wall 25 and has an opening through which the needle-attached suture thread can be removed. The lid 30 also has mating holes 31 through which the mating posts 22 can pass. When these mating posts 22 are used to join the container part 20 and the lid 30, it is preferable to form a post head 23 at the upper end of each mating post 22, the post head 23 having a slightly larger diameter than the cylindrical portion of the mating post 22, in order to prevent the attached lid 30 from easily coming off. Connecting members 27 may be provided to connect the bases of adjacent mating posts 22. The connecting members 27 not only keep the mating posts 22 vertical, but also raises the lid 30 from the bottom surface 21 of the container part 20 to provide space for storing needle-attached suture threads. Therefore, the height of each connecting member 27 extends to just below the lower end of the post head 23.

The material of the lid 30 can be paper, resin, synthetic paper made mainly from synthetic resin, or the like. However, when passing the post head 23 of the mating post 22 through the mating hole 31, there is a risk that the paper may tear, and in the case of resin, insertion may be difficult. Therefore, after passing the mating post 22 through the mating hole 31, the upper end portion of the mating post 22 may be crushed to form the post head 23. As will be described in detail later, during the thread winding operation, a claw portion is inserted between the lid 30 and the container part 22, and the claw portion slides around the thread winding tray 10. Accordingly, the lid 30 is configured to be lifted up to create a gap with respect to the container part 22 in the position where the claw portion is inserted, and in the position where the claw portion is not inserted, the lid 30 comes into contact with the container part 22.

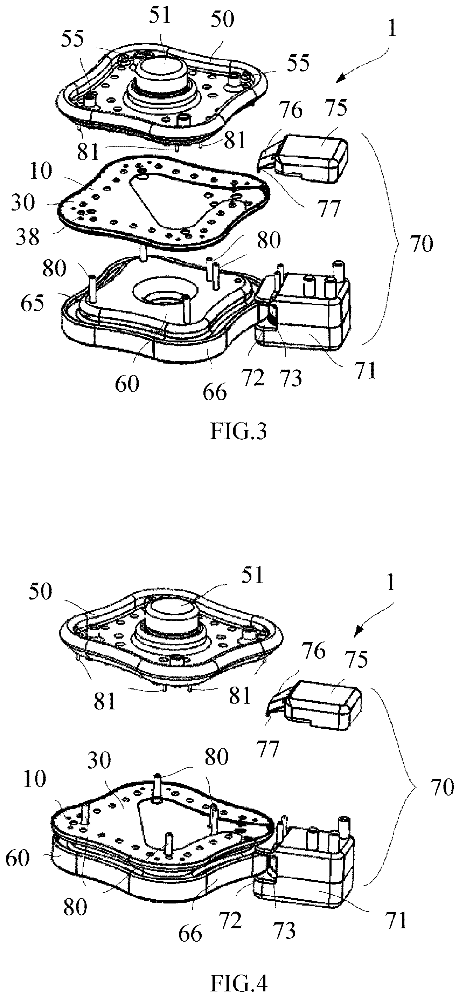

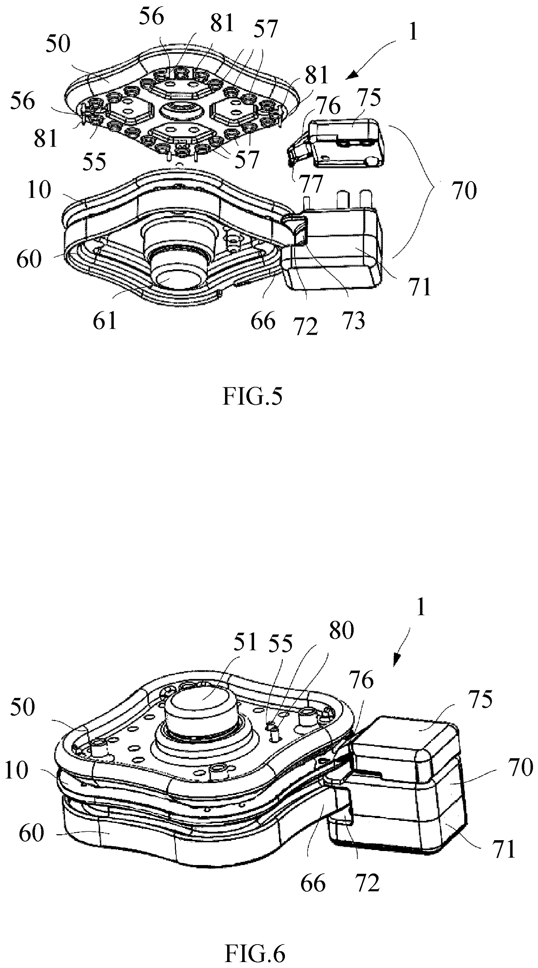

Next, the thread winding device and method for manually winding thread on such thread winding trays 10 will be described. Fig. 3 shows an example of a thread winding device and the thread winding tray according to the present invention, in a separated state. Fig. 4 shows an example of the thread winding device and the thread winding tray according to the present invention, in which the thread winding tray is mounted on a lower surface member. Fig. 5 shows an underside view of an example of the thread winding device and the thread winding tray according to the present invention, in which the thread winding tray is mounted on the lower surface member. Fig. 6 shows an example of the thread winding device according to the present invention, in which all components of the thread winding device are set in the thread winding tray. In these examples, each thread winding device is used to the corresponding thread winding tray having the rectangular shape with the constricted straight portions and the rounded corners, but these are just examples, and the shape is not limited thereto.

In order to manually wind thread onto the thread winding tray 10, a thread winding device 1 with a lower surface member 60 and a circumferential member 70 is used. In order to perform stable thread winding operation, it is preferable to use an upper surface member 50 and a plurality of coupling posts 80 connecting the upper surface member 50 and the lower surface member 60. Therefore, although the following description is based on the configuration including the upper surface member 50, the minimum thread winding operation is possible even without the upper surface member 50. Although the thread winding device of the present invention has the advantage that the thread winding operation can be done manually, it is also possible to perform the thread winding operation automatically by incorporating this thread winding device into a machine.

The upper surface member 50 and the lower surface member 60 have a planar shape that is the same as that of the thread winding tray 10. The upper surface member 50 contacts the top side of the lid 30 of the thread winding tray 10 and the lower surface member 60 contacts the bottom side of the bottom surface 21 of the container part 20, such that the thread winding tray 10 is held between them during use. The same shape as the thread winding tray 10 includes a similar shape that is about the same size as the flat shape of the thread winding tray 10. The upper surface member 50 is provided with pressing portions 57 that press the thread winding tray 10 from the top of the lid 30. The structure and shape of each pressing portion 57 can be varied to suit the thread winding tray 10, but in the case of a thread winding tray 10 in which the container part 20 and the lid 30 are joined by means of the mating posts 22, as illustrated, it is preferable to use a structure that can press the respective post heads 23 and the surrounding area of the post heads 23. Specifically, if each pressing portion 57 has a dome shape or cylindrical shape, which can encase the post heads 23, the inner surface of the dome or cylinder can hold the post heads 23 while the lid 30 can be held at the edge. This configuration ensures stability of the thread winding tray 10 during the thread winding operation using a circumference member described later, prevents the lid 30 from lifting, and prevents the lid 30 from becoming detached from the mating posts 22.

Coupling posts 80 are used to integrate the thread winding tray 10 when it is sandwiched between the upper surface member 50 and the lower surface member 60. The coupling posts 80 pass through through-holes 28 and 38 in the lid 30 and the bottom surface 21 of the container part 20, and are integrally joined by inserting the ends of the coupling posts 80 into the post insertion portions 55 and 65 in the upper surface member 50 and the lower surface member 60, respectively. In other words, it is necessary to provide through-holes 28 and 38 for the coupling posts 80 in the lid 30 and the bottom surface 21 of the container part 20 at the position where the post insertion portions 55 and 65 are provided. The coupling posts 80 can pass through the inside of the area surrounded by the mating posts 22 annularly arranged, so that they do not interfere with the thread winding operation. Since the coupling posts 80 can also be used as positioning for the thread winding tray 10, the upper surface member 50 and the lower surface member 60, two or more of them are basically required, and it is preferable to provide two to six or more of them.

The upper surface member 50 may include an upper surface rotating member 51 that can rotate freely around an axis perpendicular to the lid 30, and similarly, the lower surface member 60 may include a lower surface rotating member 61 that can rotate freely around an axis coaxial with the upper surface rotating member 51. The upper surface rotating member 51 and the lower surface rotating member 61 are about the size of a button, and when pressed between the middle finger and thumb, the thread winding tray 10 integrated with the upper surface member 50 and the lower surface member 60 can be held in one hand. Then, the integrated upper surface member 50, lower surface member 60 and thread winding tray 10 can be rotated in the hand while holding it between the middle finger and thumb. If the upper surface member 50 is not used, it is preferable that the lid 30 of the thread winding tray 10 can be held directly with the fingers.

The circumferential member 70 has: a circumferential member base 71 having an engagement portion 72 that engages a guide portion 66 provided along the outer periphery of the lower surface member 60; and a circumferential member lid 75 having a claw portion 76 that is inserted into the interior of the container part 20 from between the side wall 25 of the container part 20 and the lid 30. The circumferential member 70 is configured to become integrated by mounting the circumferential member lid 75 over the upper side of the circumferential member base 71, allowing it to move around the outer periphery of the thread winding tray 10 and the lower surface member 60. It is preferable that the circumferential member base 71 and the circumferential member lid 75 can be integrated by any method such as fitting rod-shaped posts.

The circumferential member 70 is, in principle, rotated clockwise with respect to the thread winding tray 10. From a different perspective, it may also be understood that the circumferential member 70 is fixed and the thread winding tray 10 is rotated counterclockwise. Either direction of rotation can be used, as long as the direction of rotation is predetermined. The claw portion 76 of the circumferential member lid 75 is preferably inclined toward the direction of movement to prevent it from catching on the lid 30 when the circumferential member 70 is rotated. When the thread winding tray 10 is rotated with the claw portion 76 held by upper surface member 50, the tip of the claw portion 76 can slide without leaving the bottom surface 21 of the container part 20. In other words, the upper surface member 50 can not only hold the lid 30 of the thread winding tray 10 with the pressing portion 57, but also simultaneously hold the claw portion 76 of the circumferential member 70 to stabilize the thread winding operation. Even without pressing the claw portion 76 with the upper surface member 50, it is possible to insert the claw portion 76 into the container part 20 and rotate it without floating off the bottom surface 21 by firmly holding the circumferential member 70 and rotating the thread winding tray 10. If the circumferential member lid 75 and the circumferential member base 71 can be fixed to each other, the claw portion 76 can be prevented from lifting or shifting even when the upper surface member 50 is not present, and the thread winding operation can be performed reliably. The method of fixing the circumferential member lid 75 and the circumferential member base 71 is not limited, such as snap-fitting or fixing using a separate member.

The suture thread 42 of the needle-attached suture thread 40, in which the suture needle 41 is retained to the needle retaining portion 24 of the container part 20, is set between the circumferential member base 71 and the circumferential member lid 75 before the thread winding operation, and is set so that the suture thread 42 passes through a threading portion 77 provided at the tip of the claw portion 76. The threading portion 77 has a gate-like shape with an opening underside, so that the suture thread 42 can be enclosed by the bottom surface 21 of the container part 20 and the threading portion 77 when the suture thread 42 is stretched and mounted on the top surface of the circumferential member base 71 and the circumferential member lid 75 is covered so that the suture thread 42 passes inside the threading portion 77. When the circumferential member 70 goes around the lower surface member 60, the threading portion 77 at the tip of the claw portion 76 can slide on the bottom surface 21 of the container part 20, as described above, so that the suture thread 42 does not come off the threading portion 77 during the thread winding operation. Therefore, when the suture needle 41 of the needle-attached suture thread 40 is retained to the needle retaining portion 24, the suture thread 42 passes through the threading portion 77, and the claw portion 76 is inserted between the container part 20 and the lid 30, and the thread winding tray 10 is rotated, the circumferential member 70 moves along the outer periphery of the thread winding tray 10 and the lower surface member 60, so that the suture thread 42 is wound into the thread winding tray 10 through the threading portion 77.

The circumferential member 70 can be moved by engaging the engagement portion 72 of the circumferential member base 71 with the guide portion 66 provided on the lower surface member 60, so that the circumferential member 70 does not separate from the lower surface member 60 and can perform stable thread winding operations. Also, since the circumferential member 70 is configured not to separate from the lower surface member 60, the thread winding device 1 can be made more compact than conventional thread winding devices when it is automated.

The guide portion 66 is provided in the form of a rail along the outer periphery of the lower surface member 60. Basically, it may have a guide section that is a rail in the vertical upward direction and a guide section that is a rail in the downward direction. Although only one of the guide portion 66 can have either the guide section in the vertical upward direction or the guide section in the downward direction. However, the circumferential member 70 can be moved more stably by hooking onto both the guide sections in the vertical upward direction and the downward direction. By notching a portion of the downward direction rail, it is possible to fit the engagement portion 72 into guide portion 66 having the upward direction and downward direction rails. Thus, the circumferential member 70 can engage the guide portion 66 with the claw portion 76 inserted into the interior of the container part 20.

In addition, to allow the circumferential member 70 to move smoothly, it is preferable that there be a certain clearance between the guide portion 66 and engagement portion 72. If the engagement portion 72 tightly clamps the rail-like guide portion 66, it will be difficult to move it smoothly, especially in the curved portion of the outer periphery of the lower surface member 60. However, if the clearance is too large, the angle of the circumferential member 70 with respect to the lower surface member 60 will not be stable, causing variations in the wound suture thread and making it easier for the suture thread to tangle. In this way, the operator relys on their own skill to perform the thread winding operation neatly, and the guide portion 66 becomes meaningless. In the case of a thread winding tray 10 that has concave-convex portions 26 on the inside of the side wall 25 of the container part 20, if the angle of the circumferential member 70 is not stable, the claw portion 76 will contact concave-convex portions 26 on the inside of the side wall 25, making it difficult to make a smooth movement along the periphery. Therefore, it is desirable to make the structure of the circumferential member 70 such that the angle of the circumferential member 70 with respect to the lower surface member 60 can be kept constant so that the thread can always be wound neatly without depending on the skill of the operator.

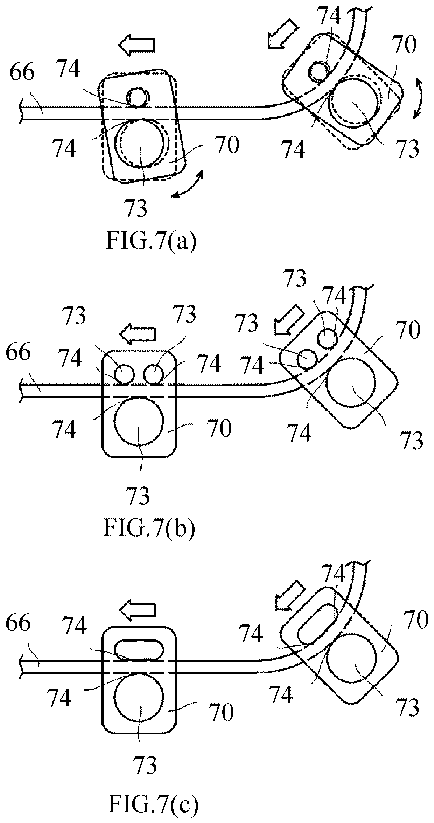

Fig. 7 illustrates the movement of the circumferential member by the number of contact points. As mentioned above, it is necessary to maintain a consistent angle of the circumferential member 70 with respect to the lower surface member 60 in order to move the circumferential member 70 smoothly and perform stable thread winding operations. Although it is desirable to increase the contact area between the guide portion 66 and the engagement portion 72, an increase in contact area hinders the smooth movement of the circumferential member 70. Therefore, to reduce the contact area, a plurality of contact points 74 are provided so that the guide portion 66 and the engagement portion 72 make point contact. The contact point 74 may have a protrusion to make contact at the tip thereof, or a roller 73. In the case of the roller 73, it is structurally more complicated, but it can move the circumferential member 70 more smoothly by rotating.

Fig. 7(a) shows a case in which one contact point 74 is provided on each of the outer and inner sides of the guide portion 66. In such a two-point contact configuration, the orientation of the circumferential member 70 with respect to the guide portion 66 tends to be unstable. Fig. 7(b) shows a case in which the guide portion 66 has one contact point 74 on the outer side and two contact points 74 on the inner side. In this configuration, the angle of the circumferential member 70 with respect to the guide portion 66 can be kept approximately constant for both the curved and straight sections. Naturally, all contact points may be rollers 73, or only one contact point on the outside may be roller 73. Fig. 7(c) shows another configuration of the three-point contact of Fig. 7(b), where the inner contact point has an elongated oval shape. With this shape, in the curved section, two contact points 74 are located inside the guide portion 66 near both ends of the oval shape, and in the straight section, the guide portion 66 can be contacted at any position of the oval shape. The outside of the guide portion 66 can be contacted by a roller 73 or the like.

In summary, if a structure is adopted in which the guide portion 66 is contacted at three or more points are established with, the angle of the circumferential member 70 with respect to the guide portion 66 can be kept approximately constant. In the part of Fig. 7(b) where the small rollers 73 and protrusions that contact the inside of the guide portion 66 are lined up, the distance between the contact points 74 and the width of the claw portion 76 may be about the same. This is because if the width of the claw portion 76 is too wide than the distance between the contact points 74 when the thread winding tray 10 and the lower surface member 60 are of the same size, the claw portion 76 will interfere with the container part 20 and the concave-convex portion 26 inside the container, making it difficult for the circumferential member 70 to slide smoothly. The above configuration of the guide portion is also effective when automating the thread winding device.

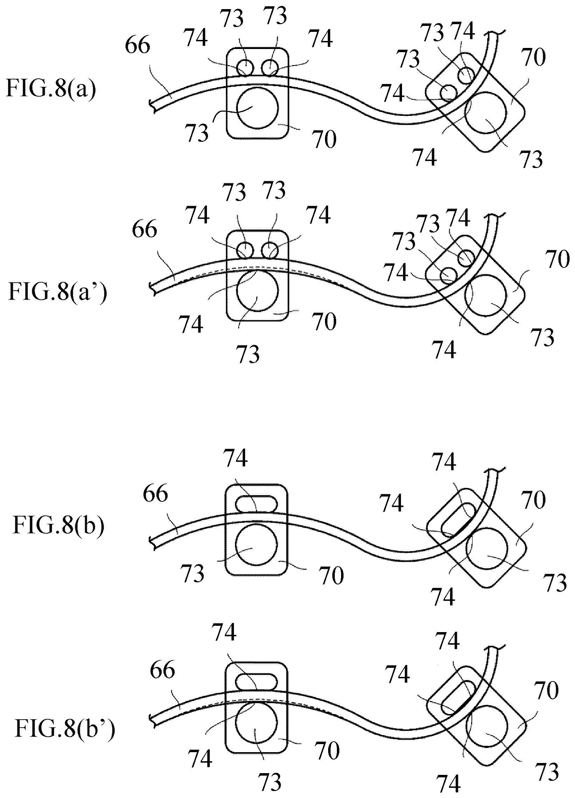

If the overall flat shape of the thread winding tray is an elliptical shape which has an outer periphery consisting of arcs and constricted straight portions (like a shape of snowboard) or a rounded corner rectangle with constricted straight sections, as shown in Figs. 1 and 2, in other words, if the outer periphery of the thread winding tray forms an annular shape with alternating right and left curves, a gap may occur between the contact points and the guide portion, so caution is required. Fig. 8 illustrates the relationship between the shape of the guide portion and the contact points of the circumferential member.

Fig. 8(a) shows a case in which the rollers 73 are provided at two points inside the guide portion 66 and one point outside the guide portion 66, and Fig. 8(b) shows elongated oval contact points 74 inside the guide portion 66 and the roller 73 outside the guide portion 66. In these configurations, there are basically three contact points 74, two on the inside of the guide portion 66 and one on the outside of the guide portion 66. However, if the thickness of the guide portion 66 is kept constant, when there are three contact points 74 at the position where the guide portion 66 bulges and curves outward, a gap occurs between the guide portion 66 and the contact points 74 at the position where the guide portion 66 is concave and curves inward, making it difficult to make contact at three points. And in such a case, there is concern that the circumferential member 70 will rattle against the guide portion 66, causing the suture thread to slip out of the container during the thread winding operation.

Therefore, as shown in Figs. 8(a') and 8(b'), it is preferable to make the guide portion 66 thicker so that three contact points 74 are maintained at locations where the guide portion 66 is curved inward. Not limited to this example, it is preferable to vary the thickness of the guide portion 66 in accordance with the curve, so that the circumferential member 70 can move stably along the guide portion 66.

In the thread winding method described above, the suture thread 42 is basically wound along the outside of the annularly arranged mating posts 22. However, when the tip of the suture thread 42 comes to the curved portion of the thread winding tray 10 or the opening of the lid 30 when finished winding, the tip of the suture thread 42 can easily protrude from the thread winding tray 10. In addition, if the suture thread 42 is wound tightly around the outer periphery of the annularly arranged mating posts 22, it may be difficult to smoothly pull out the suture thread 42 when removing the needle-attached suture thread 40 due to tangling of the suture thread 42.

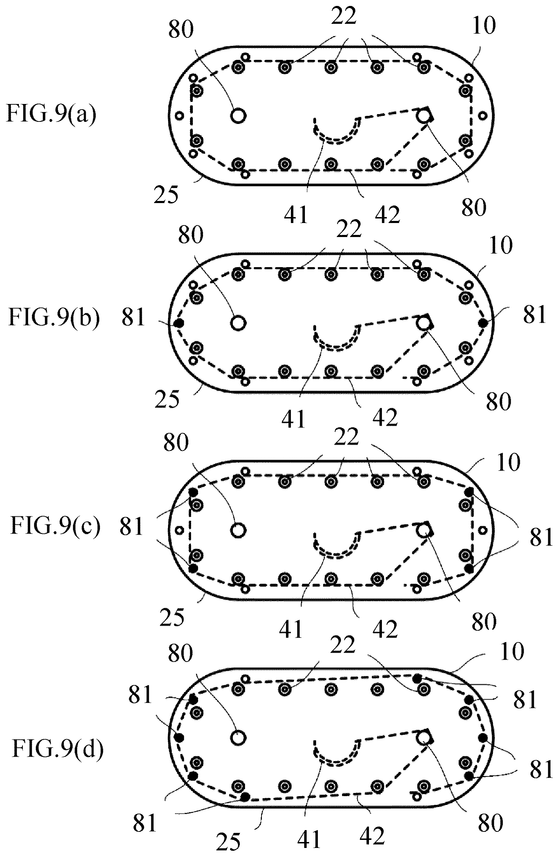

Therefore, the coupling members 81 may be to be provided outside of the annularly arranged mating posts 22. The coupling members may have any shape, including cylindrical shape, prism shape, cone shape and pyramid shape (such as rod-shaped pin). Fig. 9 illustrates the location of the coupling members. Fig. 9(a) shows a case in which suture thread 42 is wound along the outside of the annularly arranged mating posts 22, without the coupling members. Fig. 9(b) shows a case with two coupling members 81, Fig. 9(c) shows a case with four coupling members 81, and Fig. 9(d) shows a case with eight coupling members 81.

Each coupling member 81 is attached to a joining portion 56 of the upper surface member 50 and reaches the bottom surface 21 of the container part 20 through the through-portions 39 (e.g., through-holes and through-gaps) provided in the lid 30. The location of each coupling member 81 is outside the annularly arranged mating posts 22 and inside the side wall 25 of the container part 20.

The joining portions 56 on the upper surface member 50 are provided in multiple locations, and accordingly, the through-portions 39 on the lid 30 are also provided in similar locations. The thread winding operator selects some joining portions 56 to attach the coupling members 81. In other words, as shown in Figs. 9(a) to 9(d), the installation position and location can be determined arbitrarily, so the coupling members 81 may be attached to all joining portions 56, or only two joining portions 56.

At the locations where the coupling members 81 are provided, the suture thread 42 is wound around the outside of the coupling members 81, and at the locations where the coupling members 81 are not provided, the suture thread 42 is wound around the outside of the mating posts 22. In other words, at the locations where the coupling members 81 are provided, the suture thread 42 passes between each coupling member 81 and the side wall 25 closest to the coupling member 81.

Each joining portion 56 may be configured to be provided in the lower surface member 60. That is, the lower surface member 60 may have a plurality of the joining portions 56, and the coupling members 81 may be attached to all or a part of the joining portions 56. The coupling members 81 pass through the through-portions (e.g., through-holes and through-gaps) provided in the container part 20 to reach the lid 30. When the joining portions 56 are provided on the lower surface member 60, the lid 30 may or may not have the through-portions 39.

When using a strongly straight thread such as monofilament thread as suture thread 42, the suture thread 42 tends to protrude from the thread winding tray 10 because the stored suture thread 42 tends to straighten. In particular, the thread end of the suture thread 42 tends to protrude at the opening of the lid 30 or where the outer periphery of the thread winding tray 20 is bent. Therefore, if the thread end of the suture thread 42 comes to a position where it tends to protrude in the thread winding operation, it can be adjusted so that the thread end of the suture thread 42 comes to a straight portion of the thread winding tray 10 where it is difficult to protrude, by changing the attachment position and number of the coupling members 81.

In addition to monofilament threads, there are also knitted threads and other types of suture threads, which vary in thickness, length, and material, so the position where the thread ends tend to fit can vary. In addition, the length of suture thread 42 may vary during the production process, and in the case of knitted thread, some threads are cured with an adhesive to prevent the thread ends from fraying, in which the cured length may vary. However, the attachment position and number of coupling members 81 can be changed for any type of suture thread 42, so the position of the thread ends can be easily adjusted for all types of suture threads 42.

When the thread winding operation of the suture thread 42 is completed, the circumferential member 70, the upper surface member 50 and the lower surface member 60 are removed. At this time, the coupling posts 80 and coupling members 81 are pulled out of the thread winding tray 10. Then, for example, if the thread has been wound around the coupling members 81 rather than around the mating posts 22, the force that tends to straighten the suture thread 42 is likely to be weaker because the suture thread 42 would have greater slack relative to the mating posts 22. Therefore, if the coupling members 81 are placed outside of the mating posts 22, it is thought that the suture thread 42 is less likely to protrude from the thread winding tray 10. Another advantage is that the suture thread 42 can be pulled out smoothly because the suture thread 42 is wound loosely compared to when it is wound tightly on the outside of the mating posts 22.

Using the thread winding tray and thread winding device described above, it is easy to manually wind thread onto the thread winding tray and to easily adjust the thread end position of the suture thread. Specifically, by holding the circumferential member with the right hand, holding the upper surface rotating member and lower surface rotating member between the middle finger and thumb of the left hand, and rotating the thread winding tray integrated with a jig with the index finger of the left hand, the thread thread winding operator can easily perform the thread winding operation. And since the thread winding operator can perform the thread winding operation almost in the palm of the hand, the thread winder can always be in a comfortable working posture, thereby improving work efficiency. Moreover, since there is no need to introduce expensive winding machines, the economic burden can be reduced. Since this thread winding device has a simple configuration, it can be used for all kinds of suture threads and thread winding trays by preparing a thread winding device that matches the shape of the thread winding tray and the suture threads. Furthermore, especially in the case of fine suture threads, delicate handling is required, and manual operation, in which the thread handling and the thread winding rotational speed can be adjusted, is more efficient than a machine.

The thread winding device can be automated by incorporating it into a machine as well as manually. The thread winding device of the present invention can be made more compact than conventional devices because the circumferential member is configured to move along the guide portion of the lower surface member. It is also possible to limit the movement of the circumferential member and configure it to rotate the lower surface member and the thread winding tray.

### 2 Storage tray for needle-attached suture thread

### 2-1 First embodiment

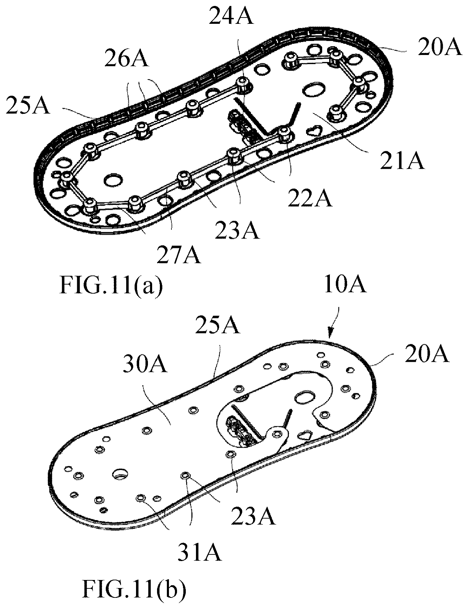

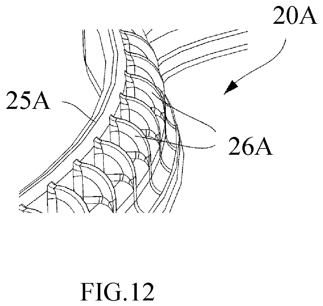

Fig. 11 shows perspective views of the storage tray of the present invention, where (a) shows the state before the lid member is attached, and (b) shows the state after the lid member is attached. Fig. 12 shows a diagram of ribs provided on the container member. The storage tray 10A is a tray for storing needle-attached suture thread with a suture needle attached to one end of the suture thread, wound in a circular shape or figure-eight pattern, and after storing the suture thread, the entire tray is enclosed and sealed with a bacteria-impermeable film or the like. Winding the suture thread in a circular shape includes winding the suture thread not only in a regular circle, but also in an oval shape or elliptical shape, according to the shape of the storage tray 10A.

The basic configuration of the storage tray 10A includes a container member 20A. The overall planar shape of the storage tray 10A may be a rectangular shape with rounded corners, an oval shape, an elliptical shape which has an outer periphery consisting of arcs and straight lines or constricted straight portions, or a rectangular shape with constricted straight portions and rounded corners. The elliptical shape which has an outer periphery consisting of arcs and constricted straight portions refers to a shape such as that shown in Fig. 11, and is, for example, similar to the shape of a snowboard. In other words, when the storage tray 10A is placed horizontally, the left and right sides are arcs, such as a semi-circle, and the upper and lower portions have an outer peripheral shape that is constricted so that they are close to each other.

The container member 20A is basically made of resin and has the shape of a shallow vessel consisting of a bottom surface 21A and a side wall 25A. The side wall 25A may be shaped to rise at right angles from the bottom surface 21A, or it may be curved in cross-section, such as a circular arc. The height of the side wall 25A may be less than 1 cm, as long as it is high enough to wind and store suture threads. The bottom surface 21A is provided with a suture needle retaining portion 24A for retaining needle-attached suture thread. Since the suture needle retaining portion 24A is only required to be able to hook and fix suture needles, it can be a retaining portion integrally formed with the container member 20A, or it can be a separate base plate that can be placed on the bottom surface 21A to pierce and fix the needle tip of the suture needle.

The storage tray 10A may further include a lid member 30A. The lid member 30A includes approximately the same shape as the container member 20A, fits inside the side wall 25A, and includes an opening for removing the stored needle-attached suture thread. Although there are various methods for joining the lid member 30A to the container member 20A, a common joining method is to have a plurality of substantially cylindrical mating posts 22A protruding from the bottom surface 21A of the container member 20A pass through the mating holes 31A in the lid member 30A. Depending on the size and shape of the container, generally, from several to about twenty mating posts 22A are annularly arranged between the center of gravity of the container member 20A and the side wall 25A.

In order to prevent the attached lid member 30A from easily coming off, it is preferable to form a post head 23A at the upper end of each mating post 22A, the post head 23A having a slightly larger diameter than the cylindrical portion of the mating post 22A. Paper, resin, or other materials can be used as the material of the lid member 30A, but synthetic paper made mainly from synthetic resin may be used because there is a risk that the paper may tear, and in the case of resin, insertion may be difficult, when passing the post head 23A of the mating post 22A through the mating hole 31A.

Connecting members 27A may also be provided to connect the bases of adjacent mating posts 22A. The connecting members 27A not only keep the mating posts 22A vertical, but also raise and support the lid member 30A from the bottom surface 21A of the container member 20A. Therefore, the height of each connecting member 27A extends to just below the lower end of the post head 23A.

For the storage tray 10A with the above configuration, a needle-attached suture thread is stored with the suture needles engaged in the suture needle retaining portion 24A and the suture threads wound approximately several to about ten times between the mating posts 22A and side wall 25A.

The inner surface of the side wall 25A of the container member 20A is provided with a plurality of ribs 26A, which are connected to both the bottom surface 21A and the side wall 25A and project toward the inside of the container member 20A. The ribs 26A are as thick as the container member 20A and their top surfaces are shaped to be lower toward the inside of the container member 20A.

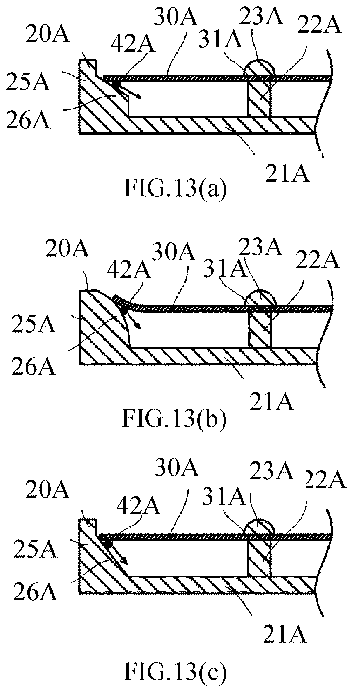

Fig. 13 shows simplified cross-sectional views of the positional relationship between the lid member and the rib, where (a) shows a case in which the rib is composed of an inclined surface and a vertical surface, (b) shows a case in which the top surface of the rib is curved, and (c) shows a case in which the top surface of the rib is straight. Fig. 13(a) shows the general shape of rib 26A. Basically, the vertical surface on the center side of the container holds suture thread 42A that bulges outward, but even if suture thread 42A comes up over the vertical surface, the inclined top surface of rib 26A allows suture thread 42A to slide toward the inside of the container member 20A and bottom surface 21A, preventing the suture thread 42A from sliding out of the container member 20A. Also, as in Figs. 13(b) and 13(c), if each rib 26A is shaped from the side view as a straight or curved line that is highest at the position connected to the side wall 25A and lowest at the position connected to the bottom surface 21A, the suture thread 42A can be moved toward the inside of container member 20A and bottom surface 21A, as in Fig. 13(a).

The shapes of these ribs 26A are only examples, and as long as the top surface of the ribs 26A is lowered toward the inside of the container member 20A, it may be a shape other than that shown as illustrated, or the top surface of each rib 26A may be a composite shape of a straight line and a curve. When the lid member 30A and the container member 20A are joined, it is preferable that the lid member 30A is in contact with the top surface of each rib 26A. In such a structure, there is no gap between the top surface of rib 26A and the lid member 30A, and the inclination of the top surface of rib 26A and the downward pressing force from the lid member 30A prevent the suture thread 42A from protruding outside the container member 20A. As shown in Fig. 13(b), the lid member 30A may be slightly bent and placed on each top surface of ribs 26A. In such a case, a stronger pressing force is applied in the direction that the lid member 30A contacts toward the ribs 26A, which is more effective in preventing the suture thread 42A from coming out.

When a particularly straight thread such as a thick monofilament thread is used as the suture thread, suture thread 42A may protrude from the storage tray 10A even if an inclination is provided on each top surface of ribs 26A, because the force of the stored suture thread to return to a straight shape. Therefore, the lid member 30A may also be provided with convex portions that press the suture thread 42A downward.

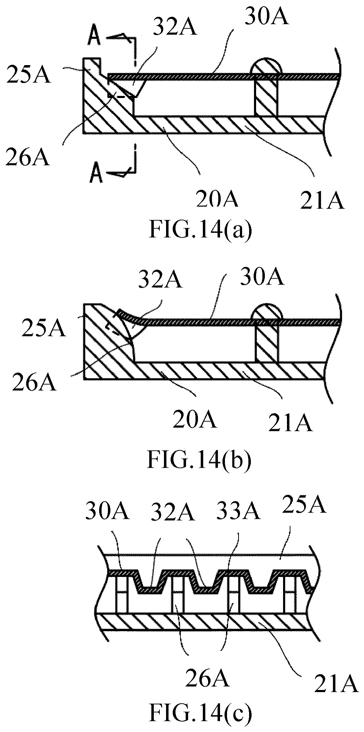

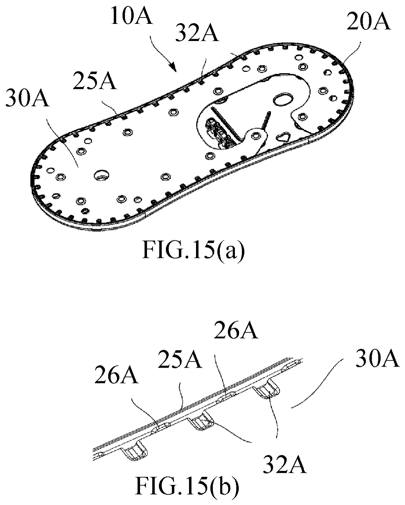

Fig. 14 shows diagrams of one or more convex portions provided on the lid member, where (a) and (b) show diagrams of a side wall in cross-sectional orientation, and (c) shows an example of an A-A cross-sectional view. Figs. 15 (a) and (d) show a perspective view of an example of the lid member with convex portions and an enlarged view thereof, respectively.

The edge of the lid member 30A is provided with a plurality of convex portions 32A projecting toward the bottom surface 21A over the entire circumference, excluding the opening. The convex portions 32A are provided at positions between adjacent ribs 26A so that the ribs 26A of the container member 20A and the convex portions 32A of the lid member 30A do not interfere when the container member 20A and the lid member 30A are joined. Each convex portion 32A may be shaped to just project downward from the lid member 30A, or it may be concave-convex as shown in Fig. 14(c). If the convex portions 32A are formed over almost the entire circumference of the edge of the lid member 30A, then a series of thickness-directional concave-convex portions 33A is formed along the edge of the lid member 30A. The term "series" means that there are no notches or slits on the edge, and the concave-convex portions 33A are connected and lined up one after the other.

When the convex portion 32A is not provided on the lid member 30A, the lid member 30A may warp so much that a gap is created between the container member 20A and the lid member 30A. In contrast, by providing the convex portions 32A on the lid member 30A, it is difficult for the lid member 30A to warp, and when it does warp, it exerts a strong pressing force on the ribs 26A, so that the convex portions 32A of the lid member 30A from above and the ribs 26A of the container member 20A from below press the suture thread 42A, respectively. This makes it very difficult for the suture thread 42A to protrude outward from the gap between the container member 20A and the lid member 30A. As long as each convex portion 32A is configured to be able to push the suture thread down to a position lower than each top surface of ribs 26A, the lid member 30A and each top surface of ribs 26A do not necessarily have to be in contact.

Since each convex portions 32A on the lid member 30A is a projecting portion in the thickness direction of the lid member 30A, the lid member 30A is easily deformed in the direction along the edge, but is difficult to deform in the radial direction (from the outer edge to the center). When using the thread insertion jig 150A as shown in Fig. 16 in the thread winding operation, the lid member 30A is easily deformed in the direction along the edge in accordance with the movement of the thread insertion jig 150A, so that the suture thread or the thread insertion jig 150A is less likely to get caught than in the conventional case where the lid member 30A is notched in the flat direction, making it less likely to interfere with the thread winding operation.

As described above, by providing the ribs on the inside of the side wall that are lowered toward the inside of the container member, and by appropriately providing convex portions on the outer periphery of the lid member in the direction of the bottom surface of the container member, needle-attached suture thread stored inside the storage tray can be prevented from protruding from the storage tray without interfering with the thread winding operation.

### 2-2 Second embodiment

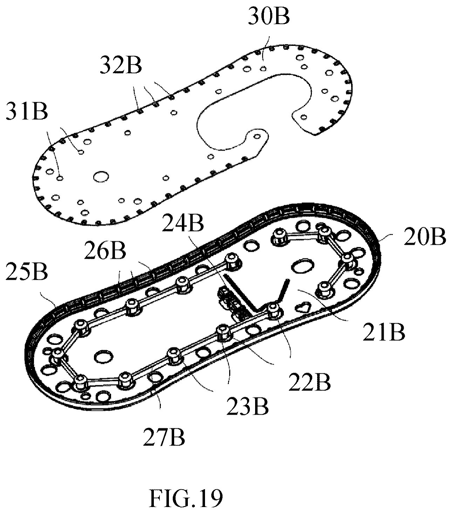

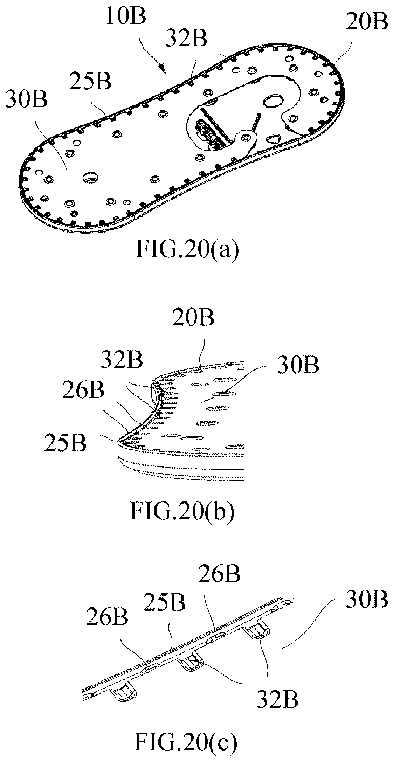

Fig. 19 shows an exploded view of the lid and container members of the storage tray of the present invention. Fig. 20 shows diagrams of the storage tray of the present invention, where (a) shows a perspective view thereof, and (b) and (c) show enlarged views of the edge of the lid member. The storage tray 10B is a tray for storing needle-attached suture thread with a suture needle attached to one end of the suture thread, and after storing the suture thread, the entire tray is enclosed and sealed with a bacteria-impermeable film or the like. The suture thread is basically wound in a circular shape, and the circular shape includes not only a regular circle but also an oval shape or an elliptical shape, depending on the shape of the storage tray 10B.

The basic configuration of the storage tray 10B has a container member 20B and a lid member 30B. The overall planar shape of the storage tray 10B may be a rectangular shape with rounded corners, an oval shape, an elliptical shape which has an outer periphery consisting of arcs and straight lines or constricted straight portions, a rectangular shape with constricted straight portions and rounded corners. The storage tray 10B illustrated in Figs. 19 and 20 has the elliptical shape which has an outer periphery consisting of arcs and constricted straight portions, like a snowboard. In other words, when the storage tray 10B is placed horizontally, the left and right sides are arcs, such as a semi-circle, and the upper and lower portions have an outer peripheral shape that is constricted so that they are close to each other.

The container member 20B has the shape of a shallow vessel with a bottom surface 21B and a side wall 25B. The side wall 25B may be shaped to rise at right angles from the bottom surface 21B, or it may be curved in cross-section, such as a circular arc. The height of the side wall 25B may be less than 1 cm, as long as it is high enough to wind and store suture threads. The bottom surface 21B is provided with a suture needle retaining portion 24B for retaining needle-attached suture thread. Since the suture needle retaining portion 24B is only required to be able to hook and fix suture needles, it can be a retaining portion integrally formed with the container member 20B, or it can be a separate base plate that can be placed on the bottom surface 21B to pierce and fix the needle tip of the suture needle.

The lid member 30B has an opening for removing the stored needle-attached suture thread, and is similar in general shape to the container member 20B. Although there are various methods for joining the lid member 30B to the container member 20B, a common joining method is to have a plurality of substantially cylindrical mating posts 22B projecting from the bottom surface 21B of the container member 20B pass through the mating holes 31B in the lid member 30B. Depending on the size and shape of the container, generally, from several to about twenty mating posts 22B are annularly arranged between the center of gravity of the container member 20B and the side wall 25B. By providing connecting members 27B that connect the bases of adjacent mating posts 22B, the connecting members 27B not only keep the mating posts 22B vertical, but also raise and support the lid member 30B from the bottom surface 21B of the container member 22B. In order to prevent the joined lid member 30B from easily coming off, it is preferable to form a post head 23B at the upper end of each mating post 22B, the post head 23B having a slightly larger diameter than the cylindrical portion of the mating post 22B. For storage tray 10B with the above configuration, the suture needles are engaged in the suture needle retaining portion 24B and needle-attached suture threads are stored with the suture threads wound several to ten times between the mating posts 22B and side wall 25B.

The material of the lid member 30B is basically a flat plate made of resin such as propylene, but it may also be made of paper or synthetic paper. The edge of the lid member 30B is provided with a plurality of groove portions 32B extending toward the bottom surface 21B over the entire circumference, excluding the opening. The groove portions 32B are formed inwardly from the edge of the lid member 30B. In other words, since the groove portions 32B are formed over almost the entire circumference of the edge of the lid member 30B, a series of thickness-directional concave-convex portions 33B is formed along the edge of the lid member 30B, in other words, the edge portion of the lid member 30B is frilled or bellows-shaped. The term "series" means that there are no notches or slits on the edge of the lid member 30B, and the concave-convex portions 33B are connected and lined up one after the other.

When the concave-convex portions 33B are not provided on the lid member 30B, the lid member 30B is a flat plate, which tends to warp near the edges, creating a gap between the container member 20B and the lid member 30B. In contrast, by providing the concave-convex portions 33B in the lid member 30B, it is difficult for the lid member 30B to warp near the edges, and the suture thread 42B stored in the container member 20B can be pressed from above by the lower surface of the groove portions 32B, so that the suture thread 42B can be prevented from protruding outside of the storage tray 10B.

The inner surface of the side wall 25B of the container member 20B may be provided with a plurality of ribs 26B, which are connected to the bottom surface 21B and the side wall 25B and project toward the inside of the container member 20B. By providing such ribs 26B, the suture thread can be stopped from spreading outward.

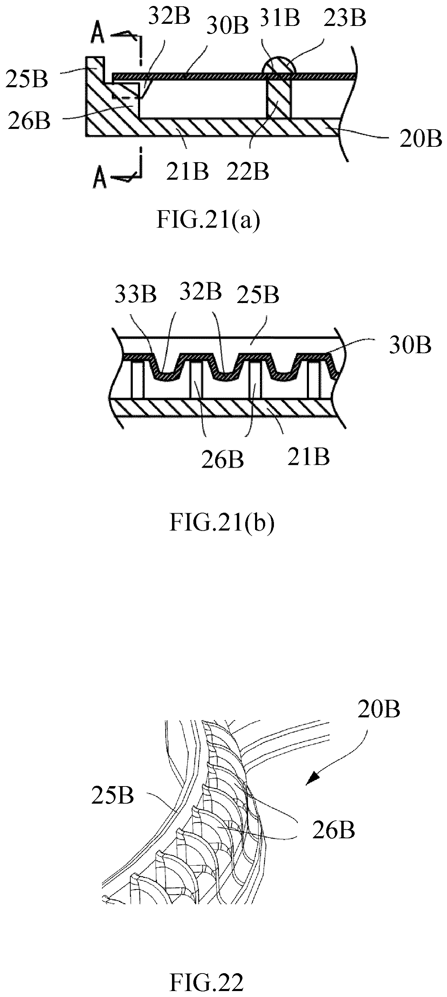

The convex portions 32B may be provided at positions between adjacent ribs 26B so that the ribs 26B of the container member 20B and the concave-convex portions 33B of the lid member 30B do not interfere when the container member 20B and the lid member 30B are joined. In other words, the concave portions of the concave-convex portions 33B may be configured to be positioned between the adjacent ribs 26B. Fig. 21 shows simplified cross-sectional views of the positional relationship between the lid member and the ribs, where (a) shows a diagram of a side wall in cross-sectional orientation, and (b) shows an example of an A-A cross-sectional view.

As shown in Figs. 21(a) and 21(b), when the groove portions 32B of the lid member 30B from above and the ribs 26B of the container member 20B from below alternately interlock with each other, it becomes very difficult for the suture thread 42B to project outward from the gap between the container member 20B and the lid member 30B. The ribs 26B protrude inwardly from the container member 20B, and similarly, the groove portions 32B are formed inwardly from the edge of the lid member 30B. The length of each groove portion 32B toward the inside of the lid member 30B may be as long as or longer than the length of the rib 26B toward the inside of the container member 20B. This is because if the length of each groove portion 32B is short, suture threads may be trapped between each top surface of ribs 26B and lid member 30B. Although it is desirable for the lid member 30B to be in contact with each top surface of the ribs 26B, the contact between the lid member 30B and each top surface of the ribs 26B is not necessarily required, as long as each lower surface of the groove portions 32B is configured to push the suture thread down to a position lower than each top surface of the ribs 26B.

The shape of each rib 26B is about the same thickness as the container member 20B, and its shape viewed from the side can be an abbreviated rectangle as shown in Fig. 21(a), or the top surface of each rib 26B can be lower toward the inside of container member 20B. When each rib 26B is an abbreviated rectangle, the inside vertical surface basically holds the suture thread 42B spreading outward, but when the suture thread 42B comes up to near the top surface of each rib 26B, if the top surface of each rib 26B is an inclined surface, then the suture thread 42B can slid toward the inside of container member 20B and bottom surface 21B, preventing the suture thread 42B from protruding out of the container member 20B.

The shape of each rib 26B viewed from the side need not be limited to an abbreviated rectangle. Fig. 22 shows an enlarged view of the rib, in which the top surface of the rib is curved. Even if the shape of each rib 26B viewed from the side is not an abbreviated rectangle, but a straight line, a curve, or a composite shape of a straight line and a curve that is highest at the position connected to side wall 25B and lowest at the position connected to bottom surface 21B, the suture thread 42B can be moved toward the inside of container member 20B and bottom surface 21B. Even in the case of such a rib 26B shape, if the groove portions 32B and ribs 26B of the lid member 30B are configured to alternately interlock with each other, it is possible to prevent the suture thread 42B from protruding outward from the gap between the container member 20B and the lid member 30B.

Since the groove portions 32B provided in the lid member 30B are projecting portions in the thickness direction of the lid member 30B, the lid member 30B is soft and easily deformable in the direction along the edge because of the concave-convex portions 33B, but it is rigid and difficult to deform in the radial direction (from the edge of the lid member to the inside direction). In other words, when a plurality of groove portions 32B are provided in the lid member 30B, the rigidity in the direction along the edge is lower than that of the flat portion of the lid member 30B, while the rigidity in the direction inward from the edge of the lid member 30B is higher than that of the flat portion of the lid member 30B. When winding thread using the thread insertion jig 150B as shown in Fig. 24, the shape of the edge portion of the lid member 30B is deformed to match the cross-sectional shape of the thread insertion jig 150B.

Fig. 23 shows illustrations comparing the shape of the lid member during the thread winding operation, where (a) shows a case without concave-convex portions, and (b) shows a case with concave-convex portions. The thread insertion jig 150B is inserted between the container member 20B, 120B and the lid member 30B, 130B, and is moved in the direction of the arrow to perform the thread winding operation. The thread insertion jig 150B is inclined in the direction of movement so that it can move smoothly without getting caught on the lid member 30B, 130B.

Fig. 23(a) shows the thread winding operation of the storage tray 110B, which does not have concave-convex portions as shown in Fig. 24, and the edge of the lid member 130B is flat, which deforms not partially but entirely, so a large gap between the lid member 130B and container member 120B on the rear side of the moving thread insertion jig 150B is likely to occur. This gap is not so large in the direction of the thickness of the thread insertion jig 150B, but is a long gap along the edge of the lid member 130B. On the other hand, Fig. 23(b) shows the thread winding operation of the storage tray 10B with the concave-convex portions 33B as shown in Fig. 20, where the concave-convex portions 33B on the edge of the lid member 30B are easily deformed by opening and closing. Thus, the gap between the lid member 130B and the container member 120B that occurs at the rear side of the moving thread insertion jig 150B is smaller than that shown in Fig. 23(a). In particular, the length of the gap can be kept shorter than with the flat lid member 130B.

Since the tip of the suture thread at the end of winding is easily caught in the gap at the rear side of the thread insertion jig 150B, the gap at the rear side of the jig 150B is closed quickly after passing through the jig 150B by providing the concave-convex portions 33B to the lid member 30B, which has a stronger effect of preventing the protrusion of the suture thread.

As described above, by appropriately providing the groove portions extending toward the bottom surface of the container member near the edge of the lid member, it is possible to prevent the protrusion of suture threads from the storage tray during the thread winding operation and after storage. Furthermore, by providing the ribs on the inside of the side wall, the synergistic effect of the groove portions and ribs can further enhance the effect of preventing the protrusion of suture threads. In addition, the concave-convex portions of the edge of the lid member formed by a plurality of the groove portions reduces the rigidity in the direction along the edge, allowing the thread winding jig to slide smoothly during the thread winding operation.

### 2-3 Third embodiment

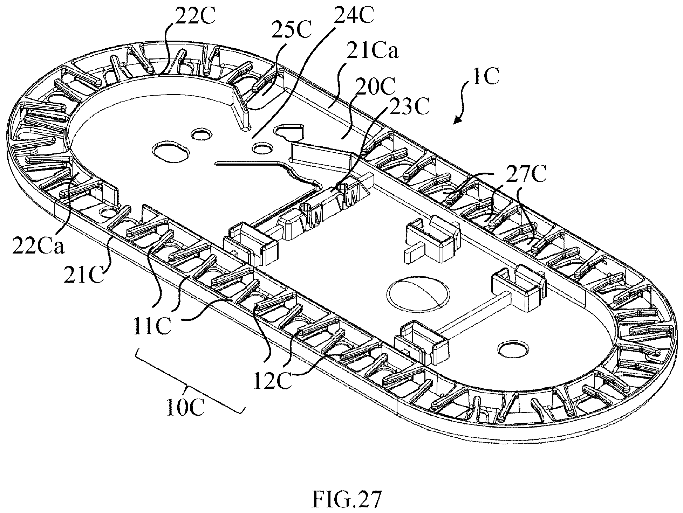

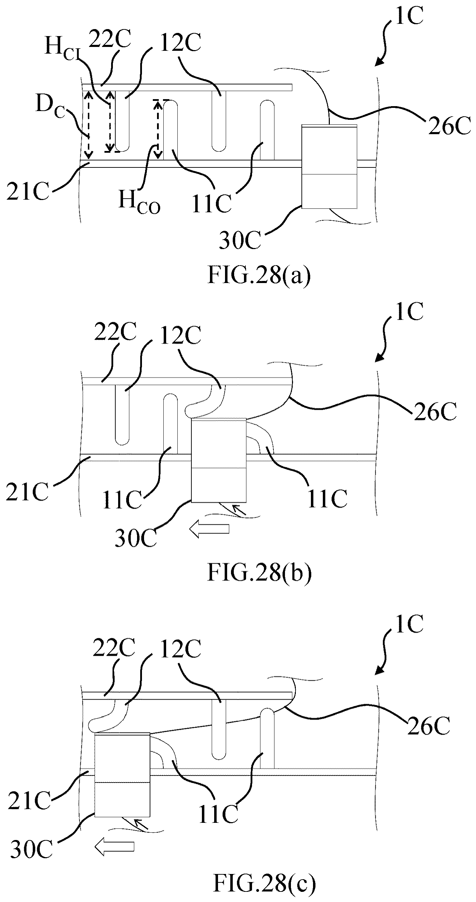

Fig. 27 shows a plan perspective view of a storage tray 1C according to the present embodiment. Figs. 28(a) to 28(c) show partial plan views of the storage tray 1C, schematically illustrating the storage of suture thread 26C in the storage tray 1C. The storage tray 1C is a tray for storing needle-attached suture thread with a suture needle (not shown) attached to one end of the suture thread 26C, wound in a circular shape, and after storing the suture thread 26C, the entire tray is enclosed and sealed with a bacteria-impermeable film or the like. Winding the suture thread 26C in a circular shape include winding the suture thread not only in a regular circle, but also in an oval shape or elliptical shape, according to the shape of the storage tray 1C.

The overall planar shape of the storage tray 1C may be a rectangular shape with rounded corners, an oval shape, an elliptical shape which has an outer periphery consisting of arcs and straight lines or constricted straight portions, or a rectangular shape with constricted straight portions and rounded corners. The elliptical shape which has an outer periphery consisting of arcs and constricted straight portions is, for example, like a snowboard (see Fig. 11). In other words, when the storage tray 1C is placed horizontally, the left and right sides are arcs, such as a semi-circle, and the upper and lower portions have an outer peripheral shape that is constricted so that they are close to each other.

The storage tray 1C is basically made of resin and has the shape of a shallow vessel with a bottom surface 20C, an outer wall 21C, and an inner wall 22C. The outer wall 21C has an outer wall surface 21Ca facing the inner wall 22C, and the inner wall 22C has an inner wall surface 22Ca facing the outer wall 21C. The wall-to-wall distance Dc between the outer wall surface 21Ca and the inner wall surface 22Ca may be constant.

The bottom surface 20C is provided with a suture needle retaining portion 23C for retaining needle-attached suture threads. Since the suture needle retaining portion 23C is only required to be able to hook and fix suture needles, it can be a retaining portion integrally formed with the storage tray 1C, or a separate base plate that can be placed on the bottom surface 20C to pierce and fix the needle tip of the suture needle.

The outer wall 21C may be shaped to rise at right angles from the outer periphery of the bottom surface 20C, or it may be curved in cross-section, such as a circular arc. The height of the outer wall 21C may be less than 1 cm, as long as it is high enough to wind and store the suture threads 26C. The inner wall 22C may be located inside the outer wall 21C and may be curved or shaped rising at right angles from the bottom surface 20C. The height of the inner wall 22C is preferably the same as the height of the outer wall 21C, but may be different.

The outer wall 21C has a plurality of outer wall arms 11C extending toward the inner wall 22C, and the inner wall 22C has a plurality of inner wall arms 12C extending toward the outer wall 21C (the plurality of outer wall arms 11C and plurality of inner wall arms 12C are also collectively referred to simply as "arms 10C"). Each of the plurality of outer wall arms 11C is spaced apart from the others. Each of the plurality of inner wall arms 12C is spaced apart from the others. The outer wall arm 11C is spaced apart from the inner wall arm 12C.

The height Hco of each outer wall arm 11C from the outer wall surface 21Ca and the height H_{CI} of each inner wall arm 12C from the inner wall surface 22Ca are shorter than the wall-to-wall distance Dc. In one embodiment, the tip in plan view of each outer wall arm 11C faces the inner wall 22C, and the tip in plan view of each inner wall arm 12C faces the outer wall 21C (if the outer wall 21C and inner wall 22C are curved surfaces, the reference surfaces are tangential planes to the outer wall 21C and inner wall 22C). The tip in plan view means the tip of the plan view projection of arm 10C on the bottom surface 20C, which may not coincide with the actual tip of arm 10C. For example, the tip in plan view of outer wall arm 11C may face the inner wall 22C even though the actual tip of outer wall arm 11C is oriented perpendicular to the bottom surface. In one embodiment, Hco is the length from the outer wall surface 21Ca to the tip of each outer wall arm 11C and H_{CI} is the length from the inner wall surface 22Ca to the tip of each inner wall arm 12C. In one embodiment, the sum of the height Hco of the outer wall arm 11C and the height H_{CI} of the inner wall arm 12C adjacent to that outer wall arm 11C is longer than the wall-to-wall distance Dc. The outer wall arm 11C may have the same length as the inner wall arm 12C or a different length.

Suture thread 26C is stored in a space 25C defined by the outer wall arms 11C, inner wall arms 12C, bottom surface 20C, outer wall 21C, and inner wall 22C. Accordingly, the outer wall arms 11C and inner wall arms 12C extend from the outer wall 21C and inner wall 22C, respectively, so as to form the space 25C capable of storing the suture thread 26C. The outer wall arms 11C and inner wall arms 12C may be parallel to the bottom surface 20C, but need not be parallel to the bottom surface 20C if the required number of suture threads 26C can be stored in the space 25C. The distance from bottom surface 20C to the arms 10C is not limited as long as the required number of suture threads 26C can be stored in space 25C using the thread insertion jig 30C (also referred to as the circumferential member) described below.

In one embodiment, each of the plurality of outer wall arms 11C is arranged alternately or partially alternately with each of the plurality of inner wall arms 12C. The expression "arranged alternately or partially alternately" refers to a situation in which two or more outer wall arms 11C and/or two or more inner wall arms 12C are arranged partially contiguously (adjacent to each other). The space between the arms 10C may be sufficient to allow the thread insertion jig 30C (also referred to as a circumferential member) to move. In one embodiment, the bottom surface 20C is provided with a plurality of slits 27C. Each slit 27C is located under each arm 10C in a plan view. The provision of slits 27C under each arm 10C enables the arms 10C to bend in the direction of the bottom surface 20C so that each tip of the arms 10C projects from the slits 27C even if the arms 10C collide with each other due to the movement of the thread insertion jig 30C, thereby avoiding stopping the thread insertion jig 30C. It is preferable that the length of each slit 27C (length in the wall-to-wall direction) is the wall-to-wall distance Dc, and the width of each slit 27C (length perpendicular to the wall-to-wall direction) is wider than the width of each corresponding arm 10C (e.g., 2 to 4 times (e.g., 2, 3 and 4 times) wider). The outer wall arms 11C and inner wall arms 12 C are flexible. The flexibility of arm 10C can be achieved by the material of arm 10C and/or the size of arm 10C (e.g., cross-sectional area relative to the longitudinal direction).

The suture thread 26C extending from the suture needle engaged in the suture needle retaining portion 23C is stored in the space 25C through the opening 24C provided in the inner wall 22C without the use of a lid member. The outer wall surface 21Ca facing the opening 24C may not be provided with an outer wall arm 11C.

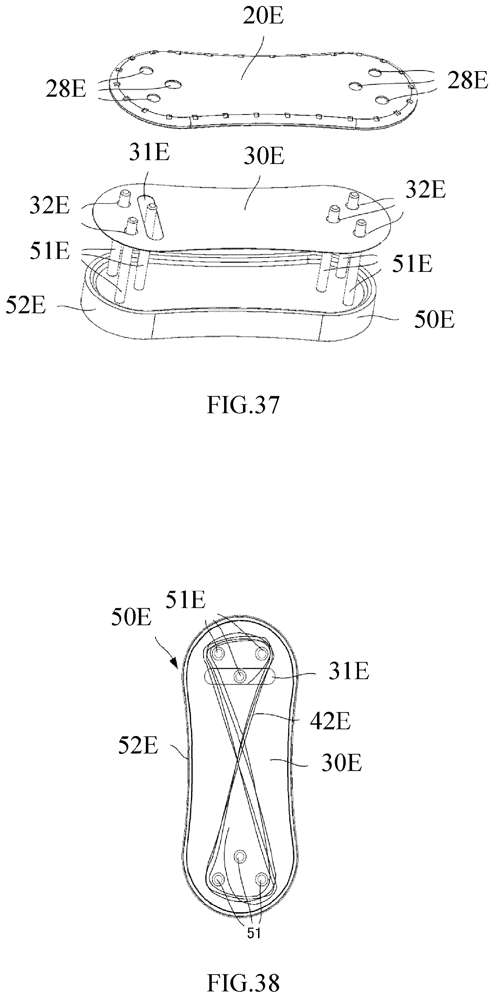

Figs. 28(a) to 28(c) show partial plan views of the storage tray 1C, schematically illustrating the storage of suture thread 26C in the storage tray 1C. The dashed line in Fig. 28(a) means an auxiliary line. The storage tray 1C can store the suture thread 26C using a thread winding jig (not shown, e.g., the thread winding jig 50E in Fig. 37) and the thread insertion jig 30C. The thread insertion jig 30C is configured to contact the outer wall arms 11C and inner wall arms 12C and to supply the suture thread 26C into the space 25C. First, the insertion jig 30C holding the suture thread 26C is placed on the outer wall 21C so that the suture thread 26C extending from the suture needle (not shown) engaged in the suture needle retaining portion 23C (not shown) enters the space 25C (Fig. 28(a)). Next, the thread insertion jig 30C is moved (in the direction of the white arrow) along the outer wall 21C (Fig. 28(b)). The outer wall arm 11C and inner wall arm 12C are restorably bent in the direction of movement of the thread insertion jig 30C by the movement of the thread insertion jig 30C. The suture thread 26C is supplied to the thread insertion jig 30C in the direction of the black arrow as the thread insertion jig 30C moves. As the thread insertion jig 30C moves, each outer wall arm 11C and each inner wall arm 12C is restored and holds the suture thread 26C in the space 25C.

Figs. 28(a) to 28(c) show a horizontal supply in which the suture thread 26C is stored in the space 25C while the thread insertion jig 30C contacts each arm 10C so that the bending of each arm 10C occurs horizontally toward the bottom surface 20C. However, the storage tray 1C according to this embodiment also allows for a vertical supply in which the suture thread 26C is stored in the space 25C while the thread insertion jig 30C contacts each arm 10C so that each arm 10C bends toward the bottom surface 20C.

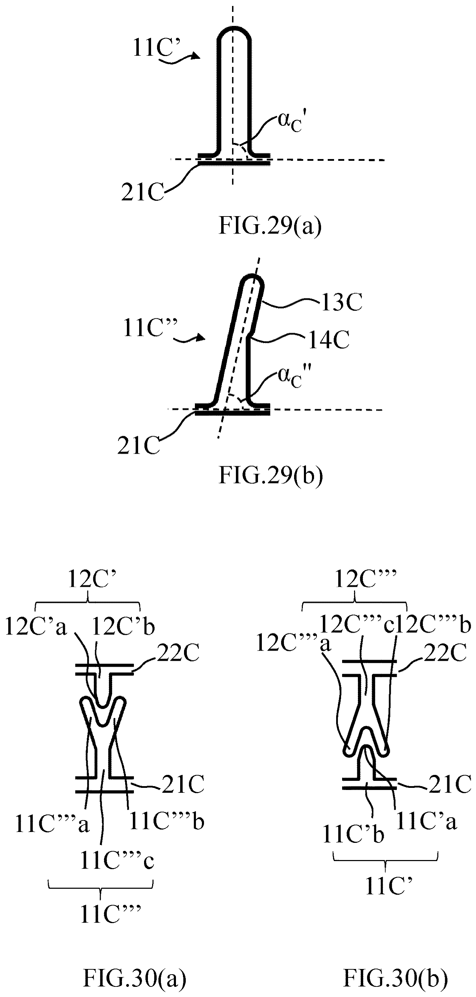

Figs. 29(a) and 29(b) are schematic plan views of examples of the outer wall arm 11C (a vertical outer wall arm 11C' and an inclined outer wall arm 11C", respectively) according to another embodiment. In Figs. 29(a) and 29(b), the dashed lines refer to auxiliary lines, and the inclination angles α_{c'} and α_{c"} refer to the angles of the vertical outer wall arm 11C' and the inclined outer wall arm 11C" relative to the outer wall 21C, respectively. Fig. 29(a) shows an example of one of the vertical outer wall arms 11C' extending perpendicularly (inclined angle α_{c'} = about 90 degrees) to the outer wall 21C. The vertical outer wall arms 11C' do not affect the direction of movement of the thread insertion jig 30C because they can bend equally in either direction. Fig. 29(b) shows an example of one of the inclined outer wall arms 11C" extending at an angle (inclination angle α_{c"} < 90 degrees) with respect to the outer wall 21C. Each inclined outer wall arm 11C" includes a neck portion 14C on a side portion 13C that is closer to the outer wall 21C, which facilitates bending. In one embodiment, the inclination angle α_{c"} may be set from 45 to 90 degrees (e.g., 45, 50, 55, 60, 65, 70, 75, 80, 85 and 90 degrees, and may be within any two angles selected from these (e.g. 50 to 85 and 55 and 80 degrees)). The neck portion 14C is preferably provided at a half arm length (half the length from the outer wall 21C to the end of the inclined outer wall arm 11C") ± 0 to 10% half arm length from the outer wall 21C. The inclined outer wall arms 11C" can easily bend in the direction of inclination and resist bending in the direction opposite to the direction of inclination, limiting the direction of movement of the thread insertion jig 30C to the direction of inclination. The inclined outer wall arms 11C" may or may not be provided with the neck portion 14C. The above examples of vertical outer wall arms 11C' and inclined outer wall arms 11C" can be applied to the inner wall arms 12C (vertical inner wall arms 12C' and inclined inner wall arms 12C') (the vertical outer wall arm 11C' and vertical inner wall arm 12C' are simply referred to as vertical arms 10C', and the inclined outer wall arms 11C" and inclined inner wall arms 12C" may simply be referred to as inclined arms 10C").

Fig. 30(a) shows a schematic plan view of an example of an outer wall arm 11C (a Y-shaped outer wall arm 11C‴) according to another embodiment. The outer wall 21C has the Y-shaped outer wall arms 11C‴ extending toward the inner wall 22C, and the inner wall 22C has vertical inner wall arms 12C' extending toward the outer wall 21C. Each Y-shaped outer wall arm 11C‴ has a first outer wall subarm 11C‴a, a second outer wall subarm 11C‴b and an outer wall arm straight body 11C‴c. One end of each outer wall arm straight body 11C‴c is connected to the outer wall 21C, and the other end of the outer wall arm straight body 11C‴c is connected to the base end of the first outer wall subarm 11C‴a and the base end of the second outer wall subarm 11C‴b, such that the Y-shaped outer wall arm 11C‴ forms a Y-shape. Each tip end of vertical inner wall arms 12C' is located between the first and second outer wall subarms 11C‴a and 11C"'b. Fig. 30(b) shows a schematic plan view of an example of an inner wall arm 12C (a Y-shaped inner wall arm 12C‴) according to another embodiment. The inner wall arm 12C may be the Y-shaped inner wall arm 12C‴ (including the first inner wall subarm 12C‴a, the second inner wall subarm 12C‴b and the inner wall arm straight body 11C‴c), and the outer wall arm 11C may be the vertical outer wall arm 11C‴ (the Y-shaped outer wall arm 11C‴ and the Y-shaped inner wall arm 12C‴ may be simply referred to as Y-shaped arms 10C‴, the first outer wall subarm 11C‴a and the first inner wall subarm 12C‴a may be simply referred to as first subarms 10C‴a, the second outer wall subarm 11C‴b and the second inner wall subarm 12C‴b may be simply referred to as second subarms 10C‴b, and the outer wall arm straight body 11C‴c and the inner wall arm straight body 110‴c may be simply referred to as arm straight bodies 10C‴c).

The outer wall 21C of this embodiment may be provided with a plurality of return-shaped members to prevent the stored suture threads 26C from coming out of the storage tray 1C. The return-shaped members project from the outer wall 21C toward the inner wall 22C and may be shorter than the height H_{CO} of the outer wall arm 11C. Each return-shaped member is preferably provided between the adjacent outer wall arms 11C.

### 2-4 Fourth embodiment

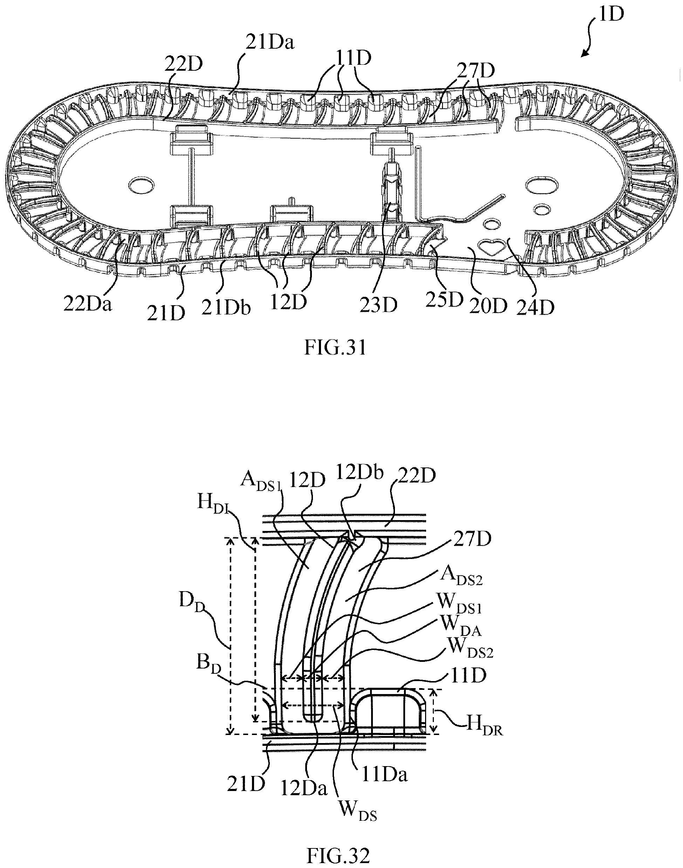

Fig. 31 shows a plan perspective view of the storage tray 1D according to the present embodiment. Fig. 32 shows a partial plan view of the storage tray 1D. In the following, the differences between the storage tray 1D and the storage trays according to the first to the third embodiments will be described.

The storage tray 1D is basically made of resin and has the shape of a shallow vessel with a bottom surface 20D, an outer wall 21D, and an inner wall 22D. The outer wall 21D has an outer wall surface 21Da facing the inner wall 22D, and the inner wall 22D has an inner wall surface 22Da facing the outer wall 21D. The wall-to-wall distance D_{D} between the outer wall surface 21Da and the inner wall surface 22Da may be constant. The bottom surface 20D has slits 27D, preferably.

A plurality of ribs 11D are provided on the outer wall surface 21Da of the outer wall 21D, each rib being connected to both the bottom surface 20D and the outer wall surface 21Da and projecting toward the inner wall 22D. The ribs 11D are about the same thickness as the storage tray 1D, and their upper surfaces are shaped to be lower toward the inner wall 22D (for details of rib 11D, see rib 26A of the first embodiment). In one embodiment, the height H_{DR} of rib 11D from the outer wall surface 21Da is shorter than the wall-to-wall distance D_{D}.

The inner wall 22D includes a plurality of inner wall arms 12D extending toward the outer wall 21D or beyond an outer wall top 21Db of the outer wall 21D. Each of the plurality of inner wall arms 12D is spaced apart from the others. In one embodiment, each inner wall arm 12D may be curved to bulge in the direction of movement of the thread insertion jig. In one embodiment, the tip end 12Da of the inner wall arm 12D may be located closer to the bottom surface 20D than its base end 12Db. In one embodiment, each inner wall arm 12D may be curved toward the bottom surface 20D. In one embodiment, the inner wall arm 12D may have a projection extending from the tip end 12Da toward the bottom surface 20D. The provision of the projection at the tip end 12Da of the inner wall arm 12D allows the thread to be hooked by the projection of the inner wall arm 12D during thread storage.

In one embodiment, the bottom surface 20D is provided with a plurality of slits 27D. Each slit 27D is located under each inner wall arm 12D in plan view. The provision of slits 27D below each inner wall arm 12D enables the inner wall arm 12D to bend in the direction of the bottom surface 20D so that the tip end 12Da of the inner wall arm 12D projects from the slits 27D even if the rib 11D and the inner wall arm 12D collide during movement of the thread insertion jig, thereby avoiding a stop of the thread insertion jig. In one embodiment, each slit 27D extends between the outer wall 21D and the inner wall 22D. In one embodiment, the width of each slit 27D (slit width W_{DS}) is, in plan view, longer than the width of the corresponding inner wall arm 12D (arm width W_{DA}) and less than the width between ribs 11D adjacent to the corresponding inner wall arm 12D or the average of the width between ribs 11D. In one embodiment, each of the plurality of slits 27D has a first slit region A_{Ds1} and a second slit region A_{Ds2} located between the inner wall arms 12D in plan view. In one embodiment, the length of the inner wall arm 12D is equal to the length of the first slit region A_{Ds1} in plan view and also equal to the length of the second slit region A_{Ds2}. The slit width W_{DS} is equal to the sum of the width of the first slit area A_{Ds1} (first slit width W_{Ds1}), the width of the second slit area A_{Ds2} (second slit width W_{DS2}) and the arm width W_{DA}. In one embodiment, the arm width W_{DA} is shorter than the sum of the first slit width W_{DS1} and the second slit width W_{DS2}. In one embodiment, the width W_{DS} of each slit 27D is preferably wider (e.g., 2 to 4 times (e.g., 2, 3 and 4 times) wider) than the arm width W_{DA} of the corresponding inner wall arm 12D.

In one embodiment, the height H_{DI} of each inner wall arm 12D from the inner wall surface 22Da is shorter than the wall-to-wall distance D_{D}. In another embodiment, the height H_{DI} of each inner wall arm 12D from the inner wall surface 22Da is longer than the wall-to-wall distance D_{D}. The sum of the height H_{DR} of each rib 11D and the height H_{DI} of inner wall arm 12D adjacent to that rib 11D is longer than the wall-to-wall distance D_{D}.

In one embodiment, each of the plurality of inner wall arms 12D is arranged without alternating or partially alternating contact with each of the plurality of ribs 11D. The expression "arranged alternately or partially alternately" refers to a situation in which two or more inner wall arms 12D and/or two or more ribs 11D are arranged partially contiguously (adjacent to each other). In one embodiment, each tip end 12Da of the inner wall arms 12D is arranged adjacent to the side walls 11Da of the ribs 11D. In one embodiment, each tip end 12Da of the inner wall arms 12D is positioned in plan view between the outer wall surface 21Da and a boundary B_{D} corresponding to the height H_{DR} of the rib 11D and along the outer wall surface 21Da.

The suture thread is shifted from the outer wall 21D toward the inner wall 22D and is stored in the space 25D defined by the inner wall arms 12D, bottom surface 20D, ribs 11 and inner wall 22D.

As described above, by providing ribs 11D on the outer wall surface 21Da, suture threads extending from suture needles retained in the suture needle retaining portion 23D can be stored in the space 25D through the opening 24D provided in the inner wall 22D without using a lid member.

### 3 Medical device storage tray

A medical device storage tray according to the present invention can store medical devices such as needle-attached suture threads wound in a figure-eight pattern or circular shape, etc. A lid member can be easily joined to the container member and is difficult to unintentionally detach.

Fig. 33 shows perspective views of the storage tray of the present invention, where (a) shows an exploded view of the lid member and the container member, and (b) shows a diagram of the lid member and the container member in a joined state. Fig. 34 shows an enlarged view of the storage tray of the present invention, where (a) shows a diagram of an edge portion of the container member, and (b) shows a diagram of the edge portion where the lid member and the container member are joined.

The basic configuration of storage tray 10E includes a container member 20E and a lid member 30E. The overall outer peripheral shape of the storage tray 10E may be a rectangular shape with rounded corners, an oval shape, an elliptical shape which has an outer periphery consisting of arcs and straight lines or constricted straight portions, or a rectangular shape with constricted straight portions and rounded corners. The elliptical shape which has an outer periphery consisting of arcs and constricted straight portions is, for example, like a snowboard. In other words, when the storage tray 10E is placed horizontally, the left and right sides are arcs, such as a semi-circle, and the upper and lower portions have an outer peripheral shape that is constricted so that they are close to each other.

The container member 20E is made of resin and has the shape of a shallow vessel including a bottom surface 21E and a side wall 25E. The cross-sectional shape of the side wall 25E can be a wall rising at right angles to the bottom surface 21E, or it can be a curved wall such as an arc as illustrated.

The lid member 30E is shaped to fit inside the side wall 25E of the container member 20E and is basically made of flat paper. Although resin or synthetic paper can be used, paper can easily be used to print product labels on its surface, eliminating the need to enclose a separate piece of paper with the product label on it when the product is made. In other words, the number of components can be reduced compared to conventional products. When absorbable thread is stored as suture thread, it can also serve as a dehumidifier, depending on the paper type. When needle-attached suture thread is stored, an opening 31E is provided in the lid member 30E, and the stored suture thread can be pulled out through the opening 31E when the needle-attached suture thread is used. The shape of the opening 31E is not particularly limited, but it is preferably a rounded slot or similar shape without corners, since the suture thread will be damaged if the lid member 30E and the suture thread rub against each other at one specific point when the suture thread is withdrawn.

The inner surface of the side wall 25E of the container member 20E may be provided with a plurality of ribs 26E, which are connected to the bottom surface 21E and the side wall 25E and project toward the inside of the container member 20E. The shape of each rib 26E may be about the same thickness as the container member 20E, and the shape viewed from the side may be an inclined surface where the top surface of each rib 26E is lower toward the inside of the container member 20E. When the lid member 30E is attached to the container member 20E, the edge of the lid member 30E basically contacts the top surfaces of the ribs 26E. By making the top surface of each rib 26E an inclined surface, the lid member 30E can be automatically moved toward the center of container member 20E to stabilize it. Furthermore, the inclined surface of each rib 26E allows the stored suture thread 42E to slide toward the inside of the container member 20E and the bottom surface 21E, preventing the suture thread 42E from protruding out of the container member 20E.

The side wall 25E is provided with not only the ribs 26E but also hook portions 27E projecting from the side wall 25E toward the inside of the container member 20E. The hook portion 27E is a member that prevents the lid member 30E from coming off by pressing the top surface of the lid member 30E when the lid member 30E and the container member 20E are joined. The hook portions 27E may be provided between adjacent ribs 26E. In other words, the ribs 26E and the hook portions 27E may be provided alternately and repeatedly around the inner circumference of the container member 20E. Since the lid member 30E is guided to the center position of the container member 20E by the inclination of each top surface of the ribs 26E, each length of the hook portions 27E hooked on the lid member 30E is constant at any position of the inner circumference of the storage tray 10E. Therefore, since the hook portions 27E exert uniform pressing force on the lid member 30E over the entire circumference, the condition in which the hook portions 27E become weakly caught at a specific position can be avoided. In this way, the lid member 30E can basically avoid being detached from the container member 20E unless the lid member 30E is intentionally removed.

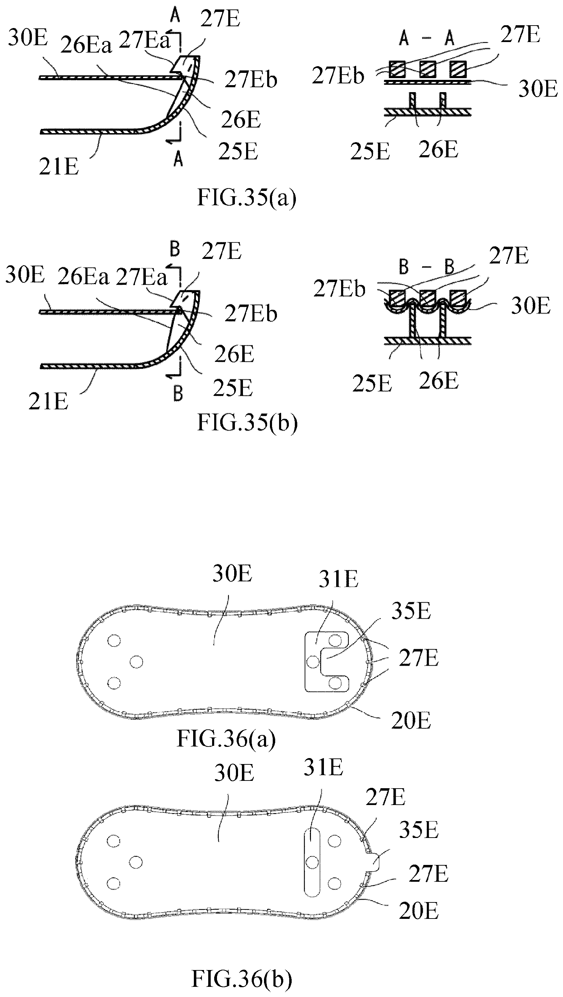

Fig. 35 shows simplified cross-sectional views and A-A (B-B) sectional views of the container member with the lid member joined, where (a) shows a case in which the top surface of the rib is an inclined surface, and (b) shows a case in which the top surface of the rib is a curved surface. When the lid member 30E is attached to the container member 20E, the lid member 30E fits between the ribs 26E and the hook portions 27E and is retained. In other words, when joining the lid member 30E to the container member 20E, the edge of the lid member 30E can be pushed from each upper side of the hook portions 27E to each lower side of the hook portions 27E. Therefore, to make it easier to push the lid member 30E, each front upper surface 27Ea of the hook portions 27E may be inclined downward toward the tip thereof. To prevent the joined lid member 30E from coming off, each bottom lower surface 27Eb of the hook portions 27E may be a horizontal surface.

In Fig. 35(a), at the edge portion of the lid member E30, each upper surface of the ribs 26E is lower than each bottom lower surface 27Eb of the hook portions E27, so the lid member 30E fits between the ribs 26E and the hook portions 27E. In such a case, the lid member 30E is basically guided to the center of the container member 20E because the edge portion of the lid member 30E is on the ribs 26E and in contact with the inclined surface.

Fig. 35(b) shows an example in which the inclined surface of rib 26E is a curved surface. In this case, at the edge of the lid member E30, each bottom lower surface 27Eb of the hook portions 27E is approximately at the same height as, or slightly higher than, each upper top surface of ribs 26E. If the lid member E30 is made of paper, the edge portion of the lid member E30 is formed to undulate in the vertical direction, as shown in the B-B sectional view, allowing it to fit between the ribs E26 and the hook portions E27. The lid member E30 is pressed from both above and below by the ribs E26 and hook portions E27, and is fixed in place.

In both cases, when the lid member 30E and the container member 20E are joined, the lid member 30E basically touches the inclined portions 26Ea of the ribs 26E. Then, even if the stored suture threads try to protrude from the storage tray 10E, since the lid member 30E is covered from above, they cannot go outside the container member 20E along the inclined portion 26Ea, and the suture threads will be returned inside. In particular, even if the suture thread is so thick that it tries to lift the lid member 30E, it is difficult for the suture thread to protrude outward because the hook portions 27E hold it in place around the entire circumference of the storage tray 10E. Even when medical devices other than suture threads are stored, it is difficult for the medical devices to protrude from the storage tray 10E if the edges of the lid member 30E are secured by the ribs 26E and the hook portions 27E.

The engaged lid member 30E is contained between the hook portions 27E and the ribs 26E, and is hard to come off unless it is intentionally removed. However, when the outer peripheral shape of the storage tray 10E has straight portions, the lid member 30E may come off when the hook portions 27E in multiple locations are released simultaneously and slide parallel to the straight portions. Therefore, by making the outer peripheral shape a series of alternating right and left curves, as in the storage tray 10E, which has an elliptical shape which has an outer periphery consisting of arcs and constricted straight portions, the lid member 30E can avoid sliding off even if the pressure of the plurality of hook portions 27E is released.

The stored needle-attached suture thread can be removed not only by pulling it out through the opening 31E in the lid member 30E, but also by removing the lid member 30E from the container member 20E and taking out the entire suture thread. In particular, in the case of barbed suture threads, it is difficult to pull them out through the opening 31E, so the lid member 30E is removed from the container member 20E and the suture thread is taken out. Also, when medical devices other than suture threads are stored, the lid member 30E is removed and taken out. If the lid member 30E is difficult to detach from the container member 20E, it will be difficult to take out the stored items, so it is desirable to have a structure that allows intentional removal.

Fig. 36 shows an example of a lid member with a tab. Fig. 36(a) shows an example in which the tab 35E is provided by modifying the shape of the opening 31E, and Fig. 36(b) shows an example in which the tab 35E is provided so as to project outward from the container member 20E.

In Fig. 36(a), by picking the tab 35E and pulling the tab 35E toward the center of the lid member 30E, the pressing forces of the plurality of hook portions 27E located near the tab 35E are removed from the lid member 30E, allowing the lid member 30E to be subsequently removed. Also in Fig. 36(b), by picking the tab 35E and moving the lid member 30E as if peeling it off, the plurality of hook portions 27E located near the tab 35E are removed from the lid member 30E, allowing the lid member 30E to be subsequently removed. These tabs 35E are designed so that the lid member 30E is difficult to remove unless it is intentionally picked and moved in the correct direction.

When storing needle-attached suture thread in the storage tray 10E, the suture thread can be easily wound into a figure-eight pattern by using a special thread winding jig. Fig. 37 illustrates how to use the thread winding jig. Fig. 38 illustrates a method of winding the suture thread in a figure-eight pattern using the thread winding jig.

The thread winding jig 50E includes a jig base 52E and a plurality of support poles 51E extending vertically from the jig base 52E. The support poles 51E are used to wind the suture thread 42E in a figure-eight pattern as well as to position the suture thread through the through-holes 28E and 32E in the lid member 30E and container member 20E, respectively.

As a specific method of thread winding, first, a suture needle of needle-attached suture thread is pulled out from the back side of the lid member 30E through the opening 31E to the front side of the lid member 30E, and the suture needle is retained on the surface of the lid member 30E. For example, the suture needle may be secured by providing a slot near the center of the lid member 30E and passing the needle through the slot, which offers the advantage of eliminating the need for a separate member to retain the suture needle. In such a case, the suture thread 42E extending from the retained suture needle is on the back side of the lid member 30E through the opening 31E.

Next, the lid member 30E is placed upside down, with its back side facing upward, and the support poles 51E are inserted through the through-holes 32E and the opening 31E of the lid member 30E, thereby placing the lid member 30E on the jig base 50E. Then, as shown in Fig. 38, the suture thread 42E is wound around the support poles 51E in a figure-eight pattern. At this time, the end of suture thread 42E is placed between the support poles 51E so that it does not protrude outside of the lid member 30E. This thread winding operation can be easily performed by hand.

Next, with the inside of the container member 20E down, the support poles 51E are inserted into the through-holes 28E, and the container member 20E is lowered along the support poles 51E to join the lid member 30E and the container member 20E. The joining method is as described in Fig. 35, and the edge portion of the lid member 30E is pushed between the hook portions 27E and the ribs 26E over the entire circumference.

Finally, when the storage tray 10E is completed by joining the lid member 30E and the container member 20E, the suture thread 42E is automatically stored inside the storage tray 10E. Therefore, the thread winding operation is completed by lifting the joined lid member 30E and container member 20E along the support poles 51E and removing them from the thread winding jig 50E. Even a lid member having a tab as shown in Fig. 36 can be used for this kind of thread winding operation.

As described above, the medical device storage tray according to the present invention has a structure in which the edge of the lid member is fixed to the container member using the ribs and hook portions provided on the side wall of the container member, making it easy to join the lid member and the container member. Furthermore, the shape of the ribs and the outer peripheral shape of the container member can be optimized to make the joining stronger, and the structure can be made difficult to unintentionally detach. In addition, since the area around the center of the container member is not used for the joining, a larger storage space can be secured. In addition, by providing a tab on the lid member, the lid member can be intentionally removed and the stored items can be easily taken out. When the stored items are suture threads, it is possible to prevent the protrusion of the suture threads from the storage tray, and furthermore, it is easy to wind the suture threads manually using a jig, and the suture threads can be securely stored in the storage tray.

### 4 Other embodiments

A configuration or part of a configuration in one embodiment can be substituted with a part of a configuration in another embodiment. For example, the thread winding tray described in "1 Thread winding tray for storing needle-attached suture threads for medical use" can be substituted for the storage tray described in "2 Storage tray for needle-attached suture thread."

The thread winding trays and storage trays disclosed in the present embodiments are not only for storing suture threads with needles but also for storing suture threads without needles.

### [Description of the Reference Numeral]

1···Thread winding device, 10···Thread winding tray, 20···Container part, 21···bottom surface, 22···Mating post, 23···Post head, 24···Needle retaining portion, 25···Side wall, 26···Protruded and recessed portion, 27···Connecting member, 28···Through hole, 30···Lid, 31···Mating hole, 38···Through-hole, 39···Through-portion, 40···Needle-attached suture thread, 41···Suture needle, 42···Suture thread, 50···upper surface member, 51···upper surface rotating member, 55···post insertion portion, 56···joining portion, 57···pressing portion, 60···lower surface member, 61···lower surface rotating member, 65···post insertion portion, 66···guide portion, 70···Circumferential member, 71···Circumferential member base, 72···Engagement portion, 73···Roller, 74···Contact point, 75···Circumferential member lid, 76···Claw portion, 77···Threading portion, 80···Coupling post, 81···Coupling member
10A···Storage tray, 20A···Container member, 21A···Bottom surface, 22A···Mating post, 23A···Post head, 24A···Needle retaining portion, 25A···Side wall, 26A···Rib, 27A···Connecting member, 30A···Lid member, 31A···Mating hole, 32A···Convex portion, 33A··· Concave-convex portion, 40A···Needle-attached suture thread, 41A···Suture needle, 42A···Suture thread, 110A···Storage tray, 120A···Container member, 121A···Bottom surface, 122A···Mating post, 125A···Side wall, 130A···Lid member, 150A···Thread insertion jig, 210A···Storage tray, 220A···Container member, 225A···Side wall, 226A···Rib, 230A···Lid member, 235A···Notch
10B···Storage tray, 20B···Container member, 21B···Bottom surface, 22B···Mating post, 23B···Post head, 24B···Needle retaining portion, 25B···Side wall, 26B···Rib, 27B···Connecting member, 30B···Lid member, 31B···Mating hole, 32B···Groove portion, 33B··· Concave-convex portion, 40B···Needle-attached suture thread, 41B···Suture needle, 42B···Suture thread, 110B···Storage tray, 120B···Container member, 121B···Bottom surface, 122B···Mating post, 125B···Side wall, 130B···Lid member, 150B···Thread insertion jig, 210B···Storage tray, 220B···Container member, 225B···Side wall, 226B···Rib, 230B···Lid member, 235B···Notch
1C···Storage tray, 10C···Arm, 10C'···Vertical arm, 10C"···Inclined arm, 10C"'··· Y-shaped arm, 10C'"a···First sub-arm, 10C"'b···Second sub-arm, 10C"'c···Arm straight body, 11C···Outer wall arm, 11C'···Vertical outer wall arm, 11C"···Inclined outer wall arm, 11C"'···Y-shaped outer wall arm, 11C"'a···First outer wall sub-arm, 11C"'b···Second outer wall sub-arm, 11C"'c···outer wall arm straight body, 12C···Inner wall arm, 12C'···Vertical inner wall arm, 12C"···Inclined inner wall arm, 12C"'···Y-shaped inner wall arm, 12C"'a···First inner wall sub-arm, 12C'"b···Second inner wall sub-arm, 12C"'c···Inner wall straight body, 13C···Side portion, 14C···Necked portion, 20C···Bottom surface, 21C···Outer wall, 21Ca···Outer wall surface, 22C···Inner wall, 22Ca···Inner wall surface, 23C··· Suture needle retaining portion, 24C···Opening, 25C...Space, 26C···Suture thread, 27C···Slit 30C···Thread insertion jig, H_{CO}···Outer wall arm height, H_{CI}···inner wall arm height, D_{C}···Distance between walls, α_{C}', α_{(C)}"···Inclination angle
1D··· Storage tray, 11D···Rib, 11Da···Side wall of rib, 12D···Inner wall arm, 12Da···Tip end of inner wall arm, 12Db···Base end of inner wall arm, 20D···Bottom surface, 21D···Outer wall, 21Da···Outer wall surface, 21Db···Outer wall top, 22D···Inner wall, 22Da···Inner wall surface, 23D···Suture needle retaining portion, 24D···Opening, 25D···Space, 27D···Slit, A_{Ds1}···First slit area, A_{Ds2}···Second slit area, B_{D}···Boundary, D_{D}···Distance between walls, H_{DR}···Rib height, H_{DI}··· Inner wall arm height, W_{DA}···Arm width, W_{DS}···Slit width, W_{DS1}···First slit width, W_{DS2}···Second slit width
10E···Storage tray, 20E···Container member, 21E···Bottom surface, 25E···Side wall, 26E···Rib, 26Ea···Inclined portion, 27E···Hook portion, 27Ea···Front upper surface, 27Eb···Bottom lower surface, 28E···Through hole, 30E···Lid member, 31E···Opening, 32E···Through hole, 35E···tab, 42E···Suture thread, 50E···Thread winding jig, 51E···Support pole, 52E···Jig base

## Claims

1. A thread winding device for storing needle-attached suture threads in a thread winding tray with a container part and an upper member, comprising:
a lower surface member supporting the container part from a bottom surface thereof; and
a circumferential member capable of moving around the lower surface member along a side surface of the lower surface member,
wherein the lower surface member comprises a guide portion on an outer periphery thereof,
the circumferential member comprises: a hook portion insertable between the upper member and the container part; and an engagement portion engageable with the guide portion, and
the circumferential member is engageable with the guide portion with the hook portion inserted into an interior of the container part.

2. The thread winding device for storing the needle-attached suture thread according to claim 1, further comprising an upper surface member capable of pressing the upper member from above.

3. The thread winding device for storing the needle-attached suture thread according to claim 2,
wherein the upper surface member comprises joining portions,
coupling members are attached to all or part of the joining portions, and
the upper member comprises through-portions allowing all the coupling members to pass through, individually.

4. The thread winding device for storing the needle-attached suture thread according to claim 1,
wherein the engagement portion comprise contact points inside the engagement portion, the contact points being capable of contacting the guide portion, and
the contact points are capable of contacting the guide portion at three or more locations.

5. The thread winding device for storing the needle-attached suture thread according to claim 4, whereing at least one of the contact points is a roller.

6. A storage tray for winding and storing needle-attached suture threads, comprising:
a container member comprising a bottom surface and a side wall,
wherein an inner surface of the side wall is provided with a plurality of ribs, each rib being connected to the bottom surface and the side wall and projecting toward an inside of the container member, and
a top surface of the rib is lowered toward the inside of the container member.

7. The storage tray for winding and storing the needle-attached suture thread according to claim 6, further comprising a lid member capable of being joined to the container member.

8. The storage tray for the needle-attached suture thread according to claim 7, wherein the top surface of the rib is a straight line, a curve, or a combination of a straight line and a curve, being highest at a point connected to the side surface and lowest at a point connected to the bottom surface.

9. The storage tray for the needle-attached suture thread according to claim 7 or 8, wherein the lid member is in contact with the top surface of the rib when the lid member and the container member are joined.

10. The storage tray for the needle-attached suture thread according to claim 7, wherein at a position between adjacent ribs, the lid member comprises one or more convex portions toward the bottom surface of the container member.

11. The storage tray for the needle-attached suture thread according to claim 10,
wherein the one or more convex portions are a plurality of convex portions, thereby forming a series of protrusions and recesses in a thickness direction along an edge of the lid member.

12. A storage tray for winding and storing needle-attached suture threads, comprising:
a bottom surface;
an outer wall provided on the bottom surface; and
an inner wall provided on the bottom surface and located inward of the outer wall,
wherein the outer wall comprises an outer wall surface facing the inner wall,
the inner wall comprises an inner wall surface facing the outer wall,
the outer wall surface of the outer wall is provided with a plurality of ribs, each rib being connected to the bottom surface and the outer wall surface and projecting toward the inner wall,
the inner wall comprises a plurality of inner wall arms extending toward the outer wall, and
a sum of height of the rib from the outer wall surface and height of the inner wall arm adjacent the rib from the inner wall surface is longer than a wall-to-wall distance between the outer wall surface and the inner wall surface.

13. The storage tray for the needle-attached suture thread according to claim 12, wherein a top surface of the rib is lowered toward the inner wall.

14. The storage tray for the needle-attached suture thread according to claim 13, wherein the top surface of the rib is a straight line, a curve, or a combination of a straight line and a curve, being highest at a point connected to the outer wall surface and lowest at a point connected to the bottom surface.

15. The storage tray for the needle-attached suture thread according to claim 12, wherein a tip end of the inner wall arm is located between the outer wall surface and a position corresponding to a height of each rib in plan view.

16. The storage tray for the needle-attached suture thread according to claim 12, wherein a tip end of the inner wall arm is located closer to the bottom surace than a base end thereof.

17. The storage tray for the needle-attached suture thread according to claim 12,
wherein the bottom surface comprises a plurality of slits,
each of the plurality of slits is located under each of the plurality of inner wall arms respectively in plan view,
each of the plurality of slits comprises a first slit region and a second slit region located between the inner wall arms in plan view,
a width of each inner wall arm is shorter than a sum of width of the first slit area and width of the second slit area.

18. A storage tray for winding and storing needle-attached suture threads, comprising:
a bottom surface;
an outer wall provided on the bottom surface; and
an inner wall provided on the bottom surface and located inward of the outer wall,
wherein the outer wall comprises a plurality of outer wall arms extending toward the inner wall,
the inner wall comprises a plurality of inner wall arms extending toward the outer wall,
the outer wall comprises an outer wall surface facing the inner wall,
the inner wall comprises an inner wall surface facing the outer wall, and
a sum of height of the outer wall arm from the outer wall surface and heigh of the inner wall arm from the inner wall surface is shorter than a wall-to-wall distance between the outer wall surface and the inner wall surface.

19. The storage tray for the needle-attached suture thread according to claim 18,
wherein the bottom surface comprises a plurality of slits,
each of the plurality of slits is located under each of the plurality of outer wall arms and the plurality of inner wall arms respectively in plan view,
a width of each of the plurality of slits is wider than the width of the corresponding outer and inner wall arms.

20. The storage tray for the needle-attached suture thread according to claim 18, wherein each of the plurality of outer wall arms is arranged alternately or partially alternately with each of the plurality of inner wall arms.

21. The storage tray for the needle-attached suture thread according to claim 18, wherein each of the plurality of outer wall arms or each of the plurality of inner wall arms is a Y-shaped arm.

22. A storage tray for winding and storing needle-attached suture threads, comprising:
a container member comprising a bottom surface and a side wall; and
a lid member capable of being joined to the container member,
wherein the lid member comprises a plurality of groove portions extending inward from an edge of the lid member, forming a series of protrusions and recesses in a thickness direction along the edge of the lid member.

23. The storage tray for the needle-attached suture thread according to claim 22,
wherein the lid member comprises the groove portions in a flat plate,
whereby a direction along the edge of the lid member has lower rigidity than a flat plate portion of the lid member, and a direction from the edge toward an interior of the lid member has higher rigidity than the flat plate portion of the lid member.

24. The storage tray for the needle-attached suture thread according to claim 22,
wherein an inner surface of the side wall is provided with a plurality of ribs, each rib being connected to the bottom surface and the side wall and projecting toward an inside of the container member, and
each groove portion formed in the lid member is located between adjacent ribs.

25. A medical device storage tray comprising:
a container member comprising a bottom surface and a side wall, and
a lid member shaped to fit inside the side wall,
wherein the inner surface of the side wall is provided with hook portions and ribs projecting toward an inside of the container member,
each rib and each hook portion are alternately repeated around an inner circumference of the container member, and
the lid member fits between the ribs and the hook portions when the lid member is attached to the container member.

26. The medical device storage tray according to claim 25,
wherein each rib comprises an inclined portion downward toward the inside of the container member, and
the lid member fits between the inclined portion and each hook portion when the lid member is attached to the container member.

27. The medical device storage tray according to claim 25 or 26, wherein an outer peripheral shape of the medical device storage tray comprises a series of alternating right and left curves.
